(19) **Europäisches Patentamt European Patent Office Office européen des brevets**

(11) **EP 4 345 506 B1**

(12) **EUROPEAN PATENT SPECIFICATION**

(45) Date of publication and mention
of the grant of the patent:
**24.06.2026 Bulletin 2026/26**

(21) Application number: **23201056.1**

(22) Date of filing: **29.09.2023**

(51) International Patent Classification (IPC):
**G01T 1/164** (2006.01)     **G01T 1/29** (2006.01)

(52) Cooperative Patent Classification (CPC):
**G01T 1/1647; G01T 1/2985**

(54) **NUCLEAR MEDICINE DIAGNOSIS APPARATUS, DATA PROCESSING METHOD, AND PROGRAM**

NUKLEARMEDIZINISCHE DIAGNOSEVORRICHTUNG, DATENVERARBEITUNGSVERFAHREN UND PROGRAMM

APPAREIL DE DIAGNOSTIC DE MÉDECINE NUCLÉAIRE, PROCÉDÉ DE TRAITEMENT DE DONNÉES ET PROGRAMME

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **30.09.2022 JP 2022159095**
**30.09.2022 JP 2022159114**
**26.09.2023 JP 2023163127**

(43) Date of publication of application:
**03.04.2024 Bulletin 2024/14**

(73) Proprietor: **Canon Medical Systems Corporation**
**Tochigi 324-0036 (JP)**

(72) Inventors:
• **KAWATA, Go**
**Otawara-shi, 324-0036 (JP)**
• **TESHIGAWARA, Manabu**
**Otawara-shi, 324-0036 (JP)**

(74) Representative: **Marks & Clerk LLP**
**15 Fetter Lane**
**London EC4A 1BW (GB)**

(56) References cited:
• **EFTHIMIOU NIKOS ET AL: "TOF-PET Image Reconstruction With Multiple Timing Kernels Applied on Cherenkov Radiation in BGO", IEEE TRANSACTIONS ON RADIATION AND PLASMA MEDICAL SCIENCES, IEEE, vol. 5, no. 5, 31 December 2020 (2020-12-31), pages 703 - 711, XP011875382, ISSN: 2469-7311, [retrieved on 20210831], DOI: 10.1109/TRPMS.2020.3048642**
• **SCHAART DENNIS R ET AL: "Time of Flight in Perspective: Instrumental and Computational Aspects of Time Resolution in Positron Emission Tomography", IEEE TRANSACTIONS ON RADIATION AND PLASMA MEDICAL SCIENCES, IEEE, vol. 5, no. 5, 27 May 2021 (2021-05-27), pages 598 - 618, XP011875318, ISSN: 2469-7311, [retrieved on 20210831], DOI: 10.1109/ TRPMS.2021.3084539**

**Description**

FIELD

Embodiments described herein relate generally to a nuclear medicine diagnosis apparatus a data processing method, and a program.

BACKGROUND

To reconstruct a Positron Emission Tomography (PET) image, the image reconstruction is performed by using a Time of Flight (ToF) kernel. In this situation, when the time width of the ToF kernel used in the image reconstruction does not match ToF capabilities of detectors, the image quality of a reconstructed image may be degraded. Accordingly, it is desirable to have the ToF capabilities of the detectors match the ToF kernel used for the image reconstruction as much as possible.

Further, possible methods for enhancing the image quality of the reconstructed image include making the capabilities of the detectors uniform so as to minimize variance in the capabilities among the detectors. However, this method may impose a large burden in terms of costs.

Efthimiou et al, "TOF-PET Image Reconstruction With Multiple Timing Kernels Applied on Cherenkov Radiation in BGO in IEEE TRANSACTIONS ON RADIATION AND PLASMA MEDICAL SCIENCES, VOL. 5, NO. 5, SEPTEMBER 2021, pages 703-711 relates to a validation and a performance evaluation of a framework that can be used to simulate and reconstruct data from TOF PET scanner geometries, modeling BGO-Cherenkov detectors. The reconstruction considers the multiple timing resolutions as provided by the Cherenkov detectors, with 25 timing kernels ranging from 180 to 263 ps. Initial reconstruction results with multiple double-Gaussian mixture TOF kernels performed *on par* with a 213-ps typical single Gaussian model in terms of contrast recovery but with better BV, due to the higher BGO sensitivity.

SUMMARY OF INVENTION

In a first aspect, a nuclear medicine diagnosis apparatus is provided as recited in claim 1.

It is also acceptable to further provide a reconstruction processing unit configured to reconstruct data corresponding to the LOR, by using the probability distribution model related to the detection time difference as a Time Of Flight (ToF) kernel.

The first light emission probability model and the second light emission probability model may each be a model related to probability density for detecting a first photon among a plurality of photons included in a detection event.

The first light emission probability model and the second light emission probability model may each be a model related to probability density for detecting a prescribed number of photons among a plurality of photons included in a detection event.

The first photon number information and the second photon number information may be specified by performing a prescribed calibration scan.

The first photon number information and the second photon number information may be specified on the basis of energy spectra obtained by the first detector and the second detector, respectively, by performing the calibration scan.

The first light emission probability model and the second light emission probability model may be based on light emission distribution function models at the first detector and at the second detector, respectively.

The first light emission probability model and the second light emission probability model may be based on expected photon number values corresponding to a detection event.

The first photon number information and the second photon number information may each include a scintillation photon number.

The first photon number information and the second photon number information may each further include a Cherenkov photon number.

The calculating unit may be configured to calculate the first light emission probability model and the second light emission probability model, while taking uncertainty of a light emission position in a scintillator into consideration.

The calculating unit may be configured: to calculate light emission probability density for each of positions in the scintillator on the basis of an attenuation coefficient; and to calculate the first light emission probability model and the second light emission probability model on the basis of a light emission distribution function and the light emission probability density.

In a second aspect, a data processing method is provided as recited in claim 19.

In a third aspect, a program is provided as recited in claim 20.

It is also acceptable to provide a reconstruction processing unit configured to reconstruct data corresponding to the Line Of Response (LOR) between the first detector and the second detector, by using a probability distribution model related to the coincidence as a Time Of Flight (ToF) kernel.

The obtaining unit may be configured to measure scintillation photon quantities, as the first photon number information and the second photon number information.

The obtaining unit may be configured to further measure Cherenkov photon quantities, as the first photon number information and the second photon number information.

The specifying unit may be configured to generate a ToF kernel between the first detector and the second detector, by adding up two or more of the probability distribution models each of which is specified for a different one of the events.

The specifying unit may be configured: to generate the first light emission probability model about the first event on the basis of the first photon number information; to generate the second light emission probability model about the second event on the basis of the second photon number information; and to specify the probability distribution model on the basis of the first light emission probability model and the second light emission probability model.

The specifying unit may be configured to generate the first light emission probability model and the second light emission probability model, on the basis of a light emission distribution function model determined for each of the events.

The first photon number information and the second photon number information measured by the obtaining unit may be obtained with respect to each of the events.

The specifying unit may be configured to calculate the first light emission probability model and the second light emission probability model while taking uncertainty of a light emission position in a scintillator into consideration.

The specifying unit may be configured: to calculate light emission probability density for each of positions in the scintillator on the basis of an attenuation coefficient; and to calculate the first light emission probability model and the second light emission probability model on the basis of a light emission distribution function and the light emission probability density.

The first light emission probability model may be a model related to probability density for detecting a prescribed number of photons among a plurality of photons included in a detection event.

BRIEF DESCRIPTION OF THE DRAWINGS

FIG. 1 is a diagram illustrating an exemplary configuration of a nuclear medicine diagnosis apparatus according to a first embodiment and a second embodiment;
FIG. 2 is a flowchart illustrating a flow of processes performed by the nuclear medicine diagnosis apparatus according to the first embodiment;
FIG. 3 is a drawing for explaining a process performed by the nuclear medicine diagnosis apparatus according to the first embodiment;
FIG. 4 is a flowchart for explaining, further in detail, an example of the process at step S300 in FIG. 2;
FIG. 5 is a chart for explaining a process performed by the nuclear medicine diagnosis apparatus according to the first embodiment;
FIG. 6 is another chart for explaining the process performed by the nuclear medicine diagnosis apparatus according to the first embodiment;
FIG. 7 is a drawing for explaining the process performed by the nuclear medicine diagnosis apparatus according to the first embodiment;
FIG. 8 is a flowchart for explaining, further in detail, another example of the process at step S300 in FIG. 2;
FIG. 9 is a drawing for explaining a process performed by the nuclear medicine diagnosis apparatus according to the first embodiment;
FIG. 10 is another drawing for explaining the process performed by the nuclear medicine diagnosis apparatus according to the first embodiment;
FIG. 11 is a chart for explaining an example of a calculation result according to the first embodiment;
FIG. 12 is a chart for explaining another example of the calculation result according to the first embodiment;
FIG. 13 is a flowchart illustrating a flow of processes performed by the nuclear medicine diagnosis apparatus according to the second embodiment;
FIG. 14 is a flowchart for explaining, further in detail, an example of the process at step S30 in FIG. 13;
FIG. 15 is a flowchart for explaining, further in detail, an example of the process at step S300 in FIG. 14;
FIG. 16 is a flowchart for explaining, further in detail, another example of the process at step S300 in FIG. 14;
FIG. 17 is a drawing for explaining a process performed by the nuclear medicine diagnosis apparatus according to the second embodiment;
FIG. 18 is a chart illustrating an example of a result of verifying a method according to the second embodiment;
FIG. 19 is another chart illustrating the example of the result of verifying the method according to the second embodiment; and

FIG. 20 is yet another chart illustrating the example of the result of verifying the method according to the second embodiment.

## DETAILED DESCRIPTION

A nuclear medicine diagnosis apparatus according to an embodiment includes processing circuitry. The processing circuitry is configured: to obtain first photon number information detected by a first detector and second photon number information detected by a second detector different from the first detector;

to calculate a first light emission probability model corresponding to the first detector on the basis of the first photon number information and a second light emission probability model corresponding to the second detector on the basis of the second photon number information; and

to specify a probability distribution model related to a detection time difference along a Line Of Response (LOR) defined by the first detector and the second detector, on the basis of the first light emission probability model and the second light emission probability model.

First Embodiment

Exemplary embodiments of a nuclear medicine diagnosis apparatus, a data processing method, and a program will be explained in detail below, with reference to the accompanying drawings.

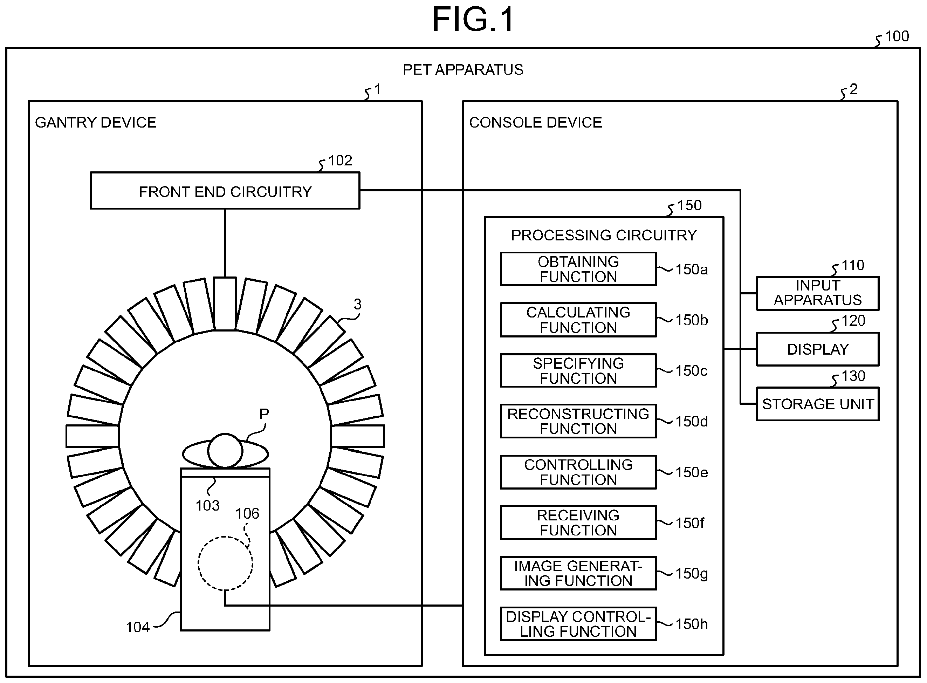

FIG. 1 is a diagram illustrating a configuration of a Positron Emission Tomography (PET) apparatus 100 serving as a nuclear medicine diagnosis apparatus according to a first embodiment. As illustrated in FIG. 1, the PET apparatus 100 according to the first embodiment includes a gantry device 1 and a console device 2. The gantry device 1 includes detectors 3, front end circuitry 102, a tabletop 103, a table 104, and a table driving unit 106.

The detectors 3 are each a detector configured to detect radiation, by detecting scintillation light (fluorescent light) which is the light re-released when a substance transitions back into a ground state after being in an excited state due to an interaction between a light emitting body and gamma rays generated from pair annihilation between positrons released from a drug administered for and accumulated in an examined subject and electrons in a surrounding tissue. Further, in an embodiment, the detectors 3 are also capable of detecting Cherenkov light. The detectors 3 are each configured to detect energy information of the radiation of the gamma rays generated from the pair annihilation between the positrons released from the drug administered for and accumulated in the examined subject and the electrons in the surrounding tissue. The plurality of detectors 3 are arranged so as to surround an examined subject (hereinafter, "patient") P in a ring formation and may form a plurality of detector blocks, for example.

Typically, each of the detectors 3 includes a scintillator crystal and a light detecting surface structured with a light detecting element.

As for a material of the scintillator crystal, it is possible to use, for example, a material suitable for generating Cherenkov light such as Bismuth Germanium Oxide (BGO) or a lead compound such as lead glass ($SiO_2$ + PbO), lead fluoride ($PbF_2$), or PWO ($PbWO_4$), for instance. Further, in other examples, it is also acceptable to use, for instance, a scintillator crystal of Lutetium Yttrium Oxyorthosilicate (LYSO), Lutetium Oxyorthosilicate (LSO), Lutetium Gadolinium Oxyorthosilicate (LGSO), or BGO, for example. The light detecting element structuring a light detecting surface 3b represents a plurality of pixels, for example. Each of the pixels is structured with a Single Photon Avalanche Diode (SPAD), for example. Further, possible configurations of the detectors 3 are not limited to the above examples. In another example, the light detecting element may be configured by using a Silicon Photomultiplier (SiPM) or a photomultiplier tube, for instance.

In another example, the scintillator crystal may be a monolithic crystal. Also, the light detecting surfaces structured with the light detecting elements may be arranged on six faces of the scintillator crystal, for example. In the first embodiment described below, an example will be first explained in which the scintillator crystals of the detectors 3 do not structure a monolithic crystal.

Further, by employing the front end circuitry 102, the gantry device 1 is configured to generate count information from output signals of the detectors 3 and to store the generated count information into a storage unit 130 of the console device 2. In this situation, the detectors 3 are divided into the plurality of blocks and are provided with the front end circuitry 102.

The front end circuitry 102 is configured to generate the count information by converting the output signals of the detectors 3 into digital data. The count information includes detection positions, energy values, and detection times of annihilation gamma rays. For example, the front end circuitry 102 is configured to identify a plurality of light detecting elements that converted scintillation light into electrical signals with mutually the same timing. Further, the front end circuitry 102 is configured to identify scintillator numbers (P) indicating the positions of the scintillators to which the annihilation gamma rays became incident. As for a means for identifying the scintillator positions at which the annihilation gamma rays became incident, the scintillator positions may be identified by performing a center-of-gravity calculation on the basis of the positions of the light detecting elements and intensities of the electrical signals. Further, when the element size corresponds between each of the scintillators and each of the light detecting elements, for example, the scintillator corresponding to a light detecting element that exhibited the largest output may be presumed as a scintillator position at which the annihilation gamma rays became incident, so as to eventually identify the scintillator position by further taking scattering between the scintillators into consideration.

Further, the front end circuitry 102 is configured to identify energy values (E) of the annihilation gamma rays that became incident to the detectors 3, by either performing an integral calculation on the intensities of the electrical signals output from the light detecting elements or measuring time (Time Over Threshold) required of the electrical signal intensities to exceed a threshold value. Further, the front end circuitry 102 is configured to identify detection times (T) at which the scintillation light from the annihilation gamma rays was detected by the detectors 3. In this situation, the detection times (T) may be absolute times or may be elapsed time periods since the start of an imaging process. As explained herein, the front end circuitry 102 is configured to generate the count information including the scintillator numbers (P), the energy values (E), and the detection times (T).

The front end circuitry 102 may be realized by using, for example, a Central Processing Unit (CPU), a Graphical Processing Unit (GPU), or circuitry such as an Application Specific Integrated Circuit (ASIC) or a programmable logic device (e.g., a Simple Programmable Logic Device (SPLD), a Complex Programmable Logic Device (CPLD), or a Field Programmable Gate Array (FPGA)). The front end circuitry 102 is an example of a front end unit.

The tabletop 103 is a bed on which the patient P is placed and is arranged over the table 104. The table driving unit 106 is configured to move the tabletop 103 under control of a controlling function 150e of processing circuitry 150. For example, the table driving unit 106 is configured to move the patient P to the inside of an imaging opening of the gantry device 1, by moving the tabletop 103.

The console device 2 is configured to control PET image taking processes by receiving operations performed on the PET apparatus 100 by an operator and to also reconstruct a PET image by using the count information acquired by the gantry device 1. As illustrated in FIG. 1, the console device 2 includes the processing circuitry 150, an input apparatus 110, a display 120, and the storage unit 130. In this situation, functional units of the console device 2 are connected together via a bus. Details of the processing circuitry 150 will be explained later.

The input apparatus 110 is a mouse, a keyboard, and/or the like used by the operator of the PET apparatus 100 for inputting various types of instructions and various types of settings and is configured to transfer the input various types of instructions and various types of settings to the processing circuitry 150. For example, the input apparatus 110 may be used for inputting an instruction to start an imaging process.

The display 120 is a monitor or the like referenced by the operator and is configured, under control of the processing circuitry 150, to display a respiratory waveform of the patient and the PET image and to display a Graphical User Interface (GUI) used for receiving the various types of instructions and the various types of settings from the operator.

The storage unit 130 is configured to store therein various types of data used in the PET apparatus 100. For instance, the storage unit 130 is configured by using a memory and may be, in an example, realized by using a semiconductor memory element such as a Random Access Memory (RAM) or a flash memory, or a hard disk, an optical disc, or the like. The storage unit 130 is configured to store therein: the count information which is the information keeping the scintillator numbers (P), the energy values (E), and the detection times (T) in correspondence with one another; coincidence information in which coincidence numbers represented by serial numbers of pieces of coincidence information are kept in correspondence with sets of count information or projection data obtained by aggregating the coincidence information; the reconstructed PET image; and/or the like.

The processing circuitry 150 includes an obtaining function 150a, a calculating function 150b, a specifying function 150c, a reconstructing function 150d, the controlling function 150e, a receiving function 150f, an image generating function 150g, and a display controlling function 150h. The functions of the obtaining function 150a, the calculating function 150b, and the specifying function 150c will be explained in detail later.

In the first embodiment and the second embodiment, processing functions performed by the obtaining function 150a, the calculating function 150b, the specifying function 150c, the reconstructing function 150d, the controlling function 150e, the receiving function 150f, the image generating function 150g, and the display controlling function 150h are stored in the storage unit 130 in the form of computer-executable programs. The processing circuitry 150 is a processor configured to realize the functions corresponding to the programs, by reading and executing the programs from the storage unit 130. In other words, the processing circuitry 150 that has read the programs has the functions illustrated within the processing circuitry 150 in FIG. 1.

Although an example will be explained with reference to FIG. 1 in which the single piece of processing circuitry (i.e., the processing circuitry 150) realizes the processing functions performed by the obtaining function 150a, the calculating function 150b, the specifying function 150c, the reconstructing function 150d, the controlling function 150e, the receiving function 150f, the image generating function 150g, and the display controlling function 150h, it is also acceptable to structure the processing circuitry 150 by combining together a plurality of independent processors, so that the functions are realized as a result of the processors executing the programs. In other words, each of the abovementioned functions may be structured as a program, so that the one piece of processing circuitry 150 executes the programs. In another example, one or more specific functions may be installed in dedicated and independent program execution circuitry.

The term "processor" used in the above explanations denotes, for example, a Central Processing Unit (CPU), a Graphical Processing Unit (GPU), or circuitry such as an Application Specific Integrated Circuit (ASIC) or a programmable logic device (e.g., a Simple Programmable Logic Device (SPLD), a Complex Programmable Logic Device (CPLD), or a Field Programmable Gate Array (FPGA)). The one or more processors are configured to realize the functions by reading and executing the programs saved in the storage unit 130.

Further, in FIG. 1, the obtaining function 150a, the calculating function 150b, the specifying function 150c, the reconstructing function 150d, the controlling function 150e, the receiving function 150f, the image generating function 150g, and the display controlling function 150h are examples of an obtaining unit, a calculating unit, a specifying unit, a reconstructing unit, a controlling unit, a receiving unit, a generating unit, and a display controlling unit, respectively. Alternatively, in place of the processing circuitry 150, the front end circuitry 102 may be in charge of processes of the calculating unit, the obtaining unit, and the specifying unit, or the like.

By employing the reconstructing function 150d, the processing circuitry 150 is configured to reconstruct the PET image on the basis of the data obtained from the front end circuitry 102. By employing the image generating function 150g, the processing circuitry 150 is configured to generate images. By employing the controlling function 150e, the processing circuitry 150 is configured to control the entirety of the PET apparatus 100, by controlling the gantry device 1 and the console device 2. For example, by employing the controlling function 150e, the processing circuitry 150 is configured to control the imaging performed the PET apparatus 100. Further, by employing the controlling function 150e, the processing circuitry 150 is configured to control the table driving unit 106. By employing the receiving function 150f, the processing circuitry 150 is configured to receive inputs of information from the user, via the input apparatus 110. Further, by employing the display controlling function 150h, the processing circuitry 150 is configured to cause the display 120 to display the PET image and other data. Further, by employing the image generating function 150g, the processing circuitry 150 is configured to generate various types of images.

Next, a background of the first embodiment will briefly be explained.

To reconstruct a PET image, the image reconstruction is performed by using a Time Of Flight (ToF) kernel. The ToF kernel denotes a probability density function of a detection event expressed as a mathematical function of a detection time difference between signals detected by two detectors.

In this situation, when the time width of the ToF kernel used in the image reconstruction does not match ToF capabilities of the detectors, the image quality of a reconstructed image may be degraded. For example, when the time width of the ToF kernel is smaller than the ToF capabilities of the detectors, estimated positions indicating radioactive substance positions after the image reconstructions may be more concentrated locally than those in an actual distribution. On the contrary, when the time width of the ToF kernel is larger than the ToF capabilities of the detectors, estimated positions indicating the radioactive substance positions after the image reconstructions may be distributed more in peripheral parts than those in the actual distribution. Accordingly, it is desirable to have the ToF capabilities of the detectors match the ToF kernel used for the image reconstruction as much as possible.

In this regard, possible methods for enhancing the image quality of the reconstructed image include making the capabilities of the detectors uniform so as to minimize variance in the capabilities among the detectors. However, making the capabilities of the detectors uniform may impose a large burden in terms of costs. To cope with this situation, in the first embodiment, a ToF kernel is calculated for each of the detectors on the basis of a light emission model. With this arrangement, it is possible to absorb the variance in the capabilities among the detectors by providing the ToF kernel for each of the detectors, and it is therefore possible to enhance the image quality.

More specifically, a nuclear medicine diagnosis apparatus according to the first embodiment includes an obtaining unit, a calculating unit, and a specifying unit. The obtaining unit is configured to obtain first photon number information detected by a first detector and second photon number information detected by a second detector different from the first detector. The calculating unit is configured to calculate a first light emission probability model corresponding to the first detector on the basis of the first photon number information and a second light emission probability model corresponding to the second detector on the basis of the second photon number information. The specifying unit is configured to specify a probability distribution model related to a detection time difference along a Line of Response (LOR) defined by the first detector and the second detector, on the basis of the first light emission probability model and the second light emission probability model.

Further, a data processing method according to the first embodiment includes: obtaining first photon number information detected by a first detector and second photon number information detected by a second detector different from the first detector; calculating a first light emission probability model corresponding to the first detector on the basis of the first photon number information and a second light emission probability model corresponding to the second detector on the basis of the second photon number information; and specifying a probability distribution model related to a detection time difference along an LOR defined by the first detector and the second detector, on the basis of the first light emission probability model and the second light emission probability model.

In addition, a program according to the first embodiment is configured to cause a computer to execute: obtaining first photon number information detected by a first detector and second photon number information detected by a second detector different from the first detector; calculating a first light emission probability model corresponding to the first detector on the basis of the first photon number information and a second light emission probability model corresponding to the second detector on the basis of the second photon number information; and specifying a probability distribution model related to a detection time difference along an LOR defined by the first detector and the second detector, on the basis of the first light emission probability model and the second light emission probability model.

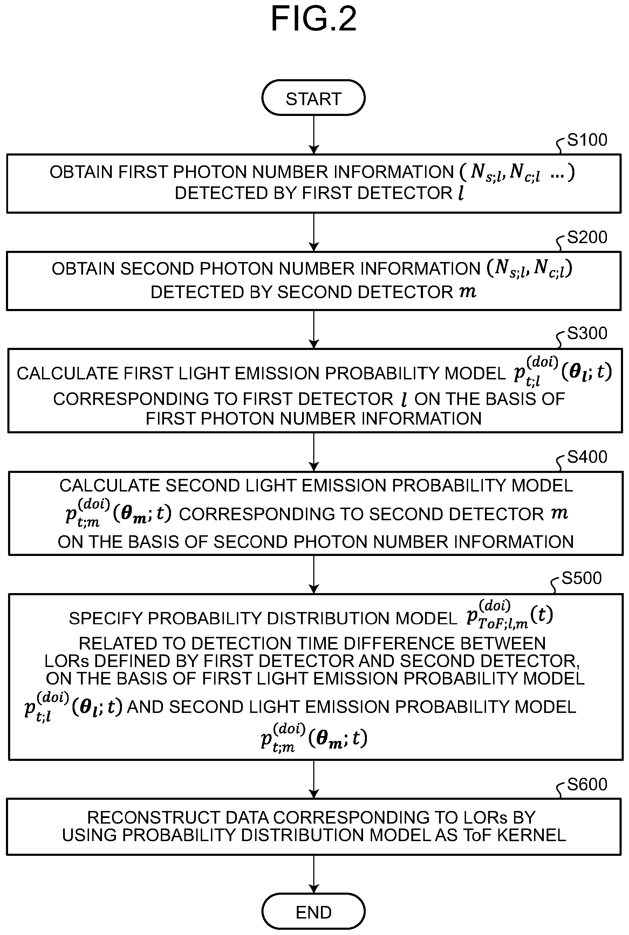

Next, processes performed by the nuclear medicine diagnosis apparatus according to the first embodiment will be explained in detail below, with reference to FIGS. 2 to 10.

FIG. 2 illustrates a flow of processes performed by the PET apparatus 100 according to the first embodiment. To simplify the explanations, the following flowchart will only describe the situation where a probability distribution model (a ToF kernel) is generated with respect to a first detector l and a second detector m. However, in image reconstruction, the processing circuitry 150 is typically configured to perform the image reconstructing process at step S600 after generating a probability distribution model with respect to every set of l and m. In that situation, the processing circuitry 150 is configured to perform the process at step S600 after repeatedly performing the processes at steps S100 through S500 with respect to every set of l and m.

At step S100, by employing the obtaining function 150a, the processing circuitry 150 is configured to obtain the first photon number information detected by the first detector l.

In this situation, the first photon number information may be, for example, a scintillation photon number $N_{s;l}$ averagely detected with respect to one standard event detected by the detector. In another example, the first photon number information may be a Cherenkov photon number $N_{C;l}$ averagely detected with respect to one standard event detected by the detector. In yet another example, the first photon number information may be the scintillation photon number $N_{s;l}$ and the Cherenkov photon number $N_{C;l}$ averagely detected with respect to one standard event detected by the detector. In other words, the first photon number information is one or more expected photon number values of the scintillation light and/or the Cherenkov light corresponding to a detection event of the detection.

The PET apparatus 100 may be configured to specify the first photon number information by performing a prescribed calibration scan. More specifically, by performing the prescribed calibration scan, the PET apparatus 100 may obtain the first photon number information and the second photon number information on the basis of an energy spectrum obtained by the first detector l.

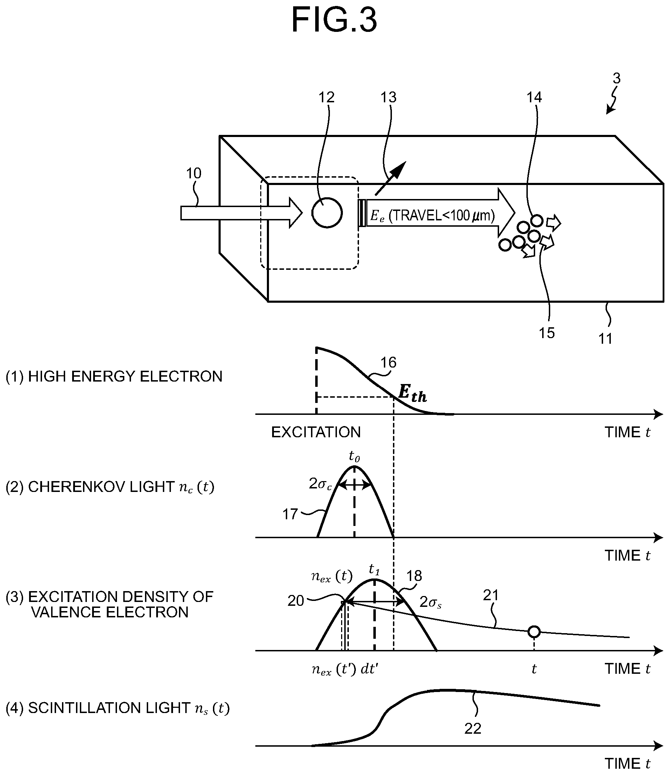

FIG. 3 schematically illustrates a light emission model in any of the detectors 3 and photon spectra obtained by the processing circuitry 150 while employing the obtaining function 150a. When a gamma ray 10 becomes incident to a scintillator 11 of a detector 3, the gamma ray 10 gives energy to the scintillator 11 in the form of photoelectric absorption or Compton scattering, and also, a high-energy electron 12 occurs. A curve 16 expresses energy $E_e$ of the high-energy electron 12 by using a mathematical function of time t. While traveling through the scintillator 11, the high-energy electron 12 loses energy, but while the energy is larger than a threshold value $E_{th}$, the high-energy electron 12 releases Cherenkov light 13 to the surroundings thereof.

A curve 17 approximates photon density $n_c(t)$ of the Cherenkov light to a Gaussian function of time t. More specifically, it is possible to express the photon density $n_c(t)$ of the Cherenkov light by using Expression (1) presented below, where $N_c$ denotes the photon number of the Cherenkov light, $\sigma_c$ denotes the half width of a Cherenkov light spectrum, and $t_0$ denotes a peak time of the Cherenkov light.

$$n_c(t) = \frac{N_C}{\sqrt{2\pi}\sigma_C} \exp\left(-\frac{(t-t_0)^2}{2\sigma_C^2}\right) \quad \cdots (1)$$

Further, while traveling, the high-energy electron 12 excites a valence electron 14 in the scintillator 11. When the excited valence electron 14 returns to a ground state, scintillation light 15 is released. A curve 18 approximates excitation density $n_{ex}(t)$ of the valence electron to a Gaussian function of time t. It is possible to express the excitation density $n_{ex}(t)$ of the valence electron by using Expression (2) presented below, where $N_s$ denotes the photon number of the scintillation light, $\sigma_s$ denotes the half width of a scintillation light spectrum, and $t_1$ denotes a peak time of the scintillation light.

$$n_{ex}(t) = \frac{N_S}{\sqrt{2\pi}\sigma_S} \exp\left(-\frac{(t-t_1)}{2\sigma_S^2}\right) \quad \cdots (2)$$

A curve 21 expresses the manner in which the valence electron excited at a time 20 transitions back to an original state. As the excited valence electron transitions back into the original state, scintillation light occurs. A curve 22 expresses photon density $n_s(t)$ of the scintillation light as a mathematical function of time t. It is possible to express the photon density $n_s(t)$ of the scintillation light by using Expression (3) presented below which uses the excitation density $n_{ex}(t)$ of the valence electron and in which $\tau_s$ denotes a relaxation constant.

$$n_S(t) = \int_{-\infty}^{t} \frac{n_{ex}(t')}{\tau_{S_i}} \exp\left(-\frac{(t-t')}{\tau_{S_i}}\right) dt' \quad \cdots (3)$$

In this situation, when a function form of the excitation density $n_{ex}(t)$ of the valence electron is assumed to be Expression (2), substituting Expression (2) into Expression (3) gives Expression (4) presented below.

$$n_S(t) = \frac{N_S}{2\tau_{S_i}} e^{\frac{\sigma_S^2}{2\tau_{S_i}^2} + \frac{t_1}{\tau_{S_i}}} \exp\left(-\frac{t}{\tau_{S_i}}\right) \left(1 + \operatorname{erf}\left(\frac{t - t_1 - \frac{\sigma_S^2}{\tau_{S_i}}}{\sqrt{2}\sigma_S}\right)\right) \quad \cdots (4)$$

In other words, the PET apparatus 100 may be configured to obtain the energy spectrum obtained by each of the first detector l and the second detector m, by performing the prescribed calibration scan, to further generate the spectra indicated by the curve 17 and the curve 22 on the basis of the energy spectra, and to obtain the first photon number information and the second photon number information on the basis of the generated spectra.

Further, because a light emission distribution function $p_{ph}(t)$ is in proportion to the sum of the photon density $n_c(t)$ of the Cherenkov light and the photon density $n_s(t)$ of the scintillation light, Expression (5) presented below holds true.

$$p_{ph}(t) \propto n_C(t) + n_S(t) \quad \cdots (5)$$

In this situation, the light emission distribution function $p_{ph}(t)$ is standardized as indicated in Expression (6) presented below.

$$\int_0^{+\infty} p_{ph}(t) = 1 \quad \cdots (6)$$

Further, at step S100, according to an embodiment not covered by the invention the processing circuitry 150 may obtain information other than the photon number information as a parameter set related to the first detector l, by employing the obtaining function 150a. In other words, the photon number information may be information that is related to the photon number that is not the photon number itself. In an example, the processing circuitry 150 may be configured, by employing the obtaining function 150a, to obtain a time resolution $\sigma_c$ of an optical sensor regarding the Cherenkov light, in place of the first photon number information or in addition to the first photon number information, as one of parameter sets $\theta_l$ obtained by the first detector l. In another example, the processing circuitry 150 may be configured, by employing the obtaining function 150a, to obtain a time resolution $\sigma_s$ of the optical sensor regarding the scintillation light, in place of the first photon number information or in addition to the first photon number information, as one of the parameter sets $\theta_l$ obtained by the first detector l. In yet another example, the processing circuitry 150 may be configured, by employing the obtaining function 150a, to obtain an excessive noise occurrence probability, as one of the parameter sets $\theta_l$ obtained by the first detector l. In this situation, the excessive noise occurrence probability denotes an occurrence probability of Avalanche Photodiode (APD) excessive noise such as optical crosstalk or afterpulsing, for example.

At step S200, by employing the obtaining function 150a, the processing circuitry 150 obtains second photon number information detected by the second detector m different from the first detector l. The second photon number information may be, for example, a scintillation photon number $N_{s;m}$ averagely detected with respect to one standard event detected by the second detector m. In another example, the second photon number information may be a Cherenkov photon number $N_{C;m}$ averagely detected with respect to one standard event detected by the second detector m, for instance. In yet another example, the second photon number information may be the scintillation photon number $N_{s;m}$ and the Cherenkov photon number $N_{C;m}$ averagely detected with respect to one standard event detected by the second detector.

Further, the PET apparatus 100 may be configured to specify the second photon number information by performing a prescribed calibration scan. More specifically, by performing the prescribed calibration scan, the PET apparatus 100 may obtain the second photon number information on the basis of an energy spectrum obtained by the second detector m.

Further, by employing the obtaining function 150a, the processing circuitry 150 may be configured to obtain a time resolution $\sigma_c$ of the optical sensor regarding the Cherenkov light, a time resolution $\sigma_s$ of the optical sensor regarding the scintillation light, an excessive noise occurrence probability, and/or the like, as a parameter set $\theta_m$ obtained by the second detector m.

At step S300, by employing the calculating function 150b, the processing circuitry 150 calculates a first light emission probability model $P_{t,l}^{(doi)}$ corresponding to the first detector l, on the basis of the first photon number information obtained at step S100. In this situation, the symbol (doi) is a symbol indicating capability of addressing both an embodiment considering uncertainty of the light emission position in the scintillator and another embodiment not considering the uncertainty. It means that, by employing the calculating function 150b, the processing circuitry 150 is configured to calculate a first light emission probability model $p_{t,l}$ in the embodiment not considering the uncertainty of the light emission position in the scintillator and is configured to calculate a first light emission probability model $p_{t,l}^{doi}$ (explained later) in the embodiment considering the uncertainty of the light emission position in the scintillator.

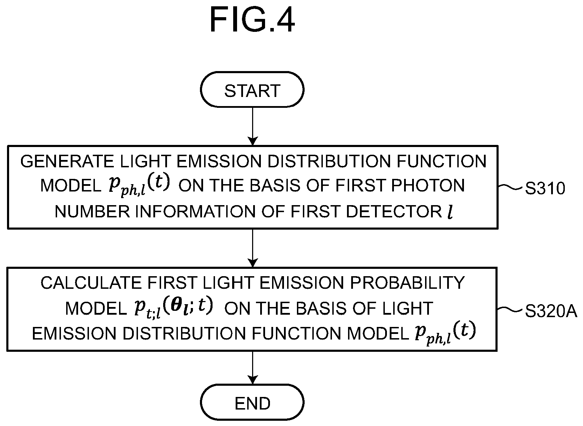

FIG. 4 illustrates an example of the process at step S300 further in detail. The flowchart in FIG. 4 is a flowchart explaining the example of the process at step S300 further in detail.

At step S310, by employing the calculating function 150b, the processing circuitry 150 generates a light emission distribution function model $p_{ph,l}(t)$ on the basis of the first photon number information of the first detector l obtained at step S100. The light emission distribution function $p_{ph}(t)$ is a distribution function indicating a probability that light emission may occur at a time t. When the light emission distribution function model $p_{ph,l}(t)$ is integrated with respect to times over the total period, the result is 1. Also, the light emission distribution function model $p_{ph,l}(t)$ is determined with respect to each of the detectors.

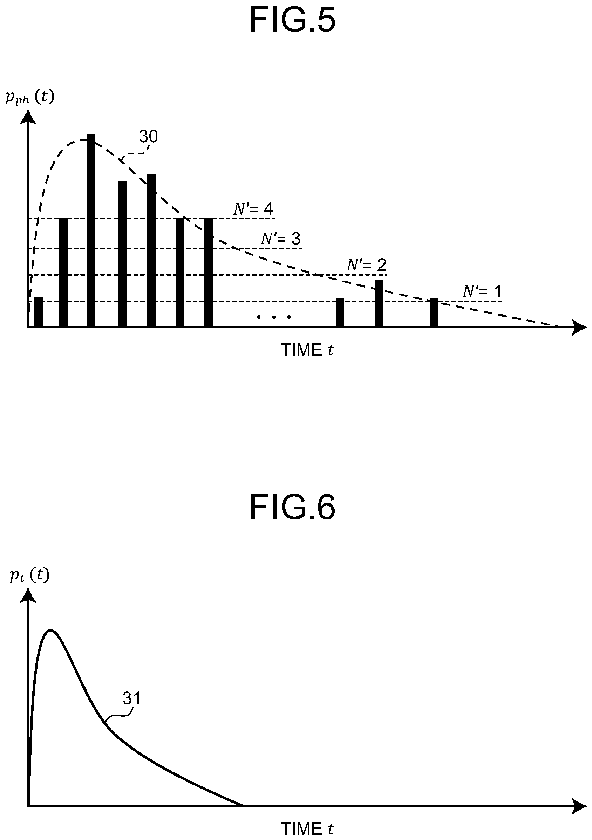

By employing the calculating function 150b, the processing circuitry 150 is configured to generate the light emission distribution function model $p_{ph,l}(t)$, by using Expressions (1), (4), (5), and (6), or the like, on the basis of the first photon number information. Because the first photon number information was obtained with respect to each of the detectors, the processing circuitry 150 is configured, by employing the calculating function 150b, to calculate the light emission distribution function model $p_{ph,l}(t)$ with respect to each of the detectors. FIG. 5 illustrates an example of the light emission distribution function $p_{ph}(t)$. In FIG. 5, a curve 30 indicates the light emission distribution function $p_{ph}(t)$. FIG. 5 also indicates threshold values (N') of the photons for defining the detection times.

Subsequently, at step S320A, by employing the calculating function 150b, the processing circuitry 150 calculates a first light emission probability model $p_{t,1}(t)$ on the basis of the light emission distribution function model $p_{ph,l}(t)$.

In this situation, the first light emission probability model $p_{t,l}(\geq N',t)$ is a model related to probability density for detecting the first N' photons among a plurality of photons included in the detection event. In other words, the first light emission probability model $p_{t,l}(\geq N',t)$ is a model related to the probability density for detecting the prescribed number of photons among the plurality of photons included in the detection event. FIG. 6 illustrates the first light emission probability model $p_{t,l}(\geq N',t)$. A graph 31 in FIG. 6 expresses a schematic form of the first light emission probability model $p_{t,l}(\geq N',t)$. In this situation, the first light emission probability model $p_{t,l}(\geq N',t)$ is determined for each of the detectors. In the following sections, the subscripts of the detectors may be omitted as appropriate, when necessary.

It is possible to calculate the first light emission probability model $pt(\geq N',t)$, which is the probability density for detecting the first N' photons among the plurality of photons included in the detection event, in the following manner.

To begin with, an equation is derived about a probability $P'(\geq N',t)$ that only photons no more than N' may have been detected by the time t. This equation indicates the probability that only N'-1 photons at most may have been detected by the time t, which can be expressed by using Expression (7) presented below, while using a probability $Pd(k,t)$ that k photons may have been detected by the time t.

$$P'(\geq N', t) = \sum_{k < N'} P_d(k, t) \quad \cdots (7)$$

It is possible to express the probability that k photons may have been detected by the time t, while using a differential format as indicated in Expression (8) presented below, by evaluating the photon detection event with a binomial distribution.

$$\frac{d(P_d(k,t)e^{NP_{ph}(t)})}{dt} = NP_d(k-1,t)e^{NP_{ph}(t)}p_{ph}(t) \quad \cdots (8)$$

When the differential equation is sequentially solved with respect to k = 0, 1, 2 and so on, Expression (9) presented below holds true inductively.

$$P_d(k,t) = \frac{N^k}{k!} F^k(t) e^{-NP_{ph}(t)} \quad \cdots (9)$$

On the basis of Expression (7), it is possible to express the probability P' ($\geq$ N',t) that no more than N' photons may have been detected by the time t, by using Expression (10) presented below.

$$P'(\geq N', t) = \frac{N^k}{k!} F^k(t) e^{-NP_{ph}(t)} \quad \cdots (10)$$

By substituting the above into Expression (8), it is possible to express the first light emission probability model pt ($\geq$ N',t), which is the probability density for detecting the first N' photons among the plurality of photons included in the detection event, by using Expression (11) presented below.

$$p_T(\geq N',t) = -\frac{dP'(\geq N', t)}{dt} = \frac{N^{N'}}{(N'-1)!} F^{N'-1}(t) p_{ph}(t) e^{-NP_{ph}(t)} \; for \; N' \geq 1 \quad \cdots (11)$$

In the above expression, the mathematical function F(t) is a cumulative distribution function of the light emission distribution function $p_{ph}(t)$ and can be expressed by using Expression (12) presented below.

$$F(t) \equiv \int_0^t p_{ph}(t') dt' \quad \cdots (12)$$

To simplify the explanations, the embodiment below will use an example of a probability distribution evaluating the time at which a photon was detected for the first time, i.e., N' = 0. In other words, pt(t) denotes a first light emission probability model that expresses probability density for detecting the first photon and can be expressed by using Expression (13) presented below.

$$p_t(t) = N p_{ph}(t) \exp(-NF(t)) \quad \cdots (13)$$

In other words, by employing the calculating function 150b, the processing circuitry 150 is able to calculate the first light emission probability model pt(t) on the basis of the light emission distribution function $p_{ph}(t)$, by using Expressions (12) and (13). In this manner, by employing the calculating function 150b, the processing circuitry 150 is configured to calculate the first light emission probability model pt(t), on the basis of the first photon number information and the light emission distribution function model $p_{ph}(t)$ of the first detector, by substituting the light emission distribution function model $p_{ph}(t)$ of the first detector into Expressions (12) and (13) and further substituting the N on the right-hand side of Expression (13) with the first photon number information and evaluating the right-hand side of Expression (13).

In this situation, it is possible to determine the light emission distribution function $p_{ph}(t)$ and the first light emission probability model pt(t) described above, for each of the detectors. Accordingly, by explicitly indicating the subscripts of the detectors, it is also possible to rewrite Expression (13) as Expression (14) presented below.

$$p_{t;l}(t) = (N_{s;l} + N_{c;l}) p_{ph;l}(t) \exp\left(-(N_{s;l} + N_{c;l}) \int_0^t p_{ph;l}(t') dt'\right) \quad \cdots (14)$$

In the above expression, $p_{t;l}(t)$ denotes the first light emission probability model of the detector l; $p_{ph;l}(t)$ denotes the light emission distribution function of the detector l; $N_{s;l}$ denotes the photon number of the scintillation light of the detector l; and $N_{c;l}$ denotes the photon number of the Cherenkov light of the detector l. As observed from this expression, the first light emission probability model $p_{t,l}(t)$ is based on the expected photon number values $N_{s;l}$ and $N_{c;l}$ corresponding to the detection event.

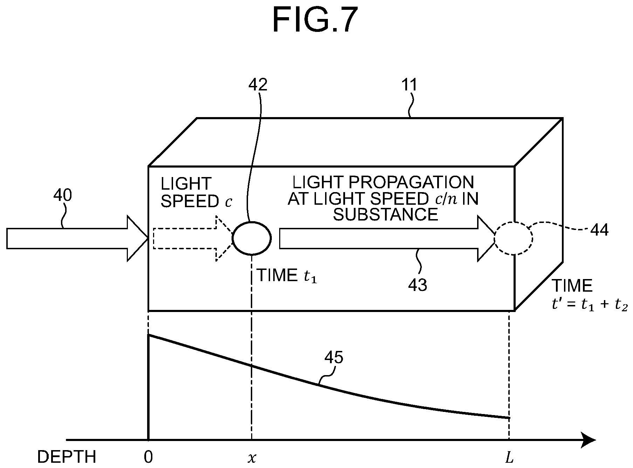

When calculating the first light emission probability model, the processing circuitry 150 is also capable of performing the calculation while taking the uncertainty of the light emission position in the scintillator into consideration, by employing the calculating function 150b. For example, let us discuss a situation in which, as illustrated in FIG. 7, a gamma ray 40 emits light at a point 42 which is at a depth x in the scintillator 11 so that, as indicated with an arrow 43, scintillation light propagates through a medium having a refractive index n at light speed c/n in the substance and reaches an end 44 of the scintillator 11. In this situation, because the time from when the gamma ray 40 becomes incident to the scintillator, to when the gamma ray 40 reaches the end of the scintillator 11 varies depending on the light emission position in the scintillator, the time at which a signal is detected also varies.

In other words, time $t_1$ it takes for the gamma ray 40 to reach the point at the depth x since the gamma ray 40 became incident to the scintillator 11 can be expressed by using Expression (15) presented below.

$$t_1 = \frac{x}{c} \quad \cdots (15)$$

Further, time $t_2$ it takes for the light emitted at the point 42 to travel from the depth x to reach the end of the scintillator 11 can be expressed by using Expression (16) presented below.

$$t_2 = \frac{L - x}{\frac{c}{n}} \quad \cdots (16)$$

Accordingly, time t' it takes for the scintillation light to reach the end of the scintillator 11 since the gamma ray 40 became incident to the scintillator 11 can be expressed by using Expression (17) presented below.

$$t' = t_1 + t_2 = \frac{nL - (n-1)x}{c} \quad \cdots (17)$$

From expression (17), it is understood that the time at which a signal is detected varies depending on the light emission position x in the scintillator.

When we address the notion of taking the light emission position x in the scintillator into consideration as explained above, Expression (18) presented below holds true.

$$p_{t;l}^{doi}(t) = (N_{s;l} + N_{c;l})p_{ph;l}^{doi}(t) \exp\left(-(N_{s;l} + N_{c;l})\int_0^t p_{ph;l}^{doi}(t')\,dt'\right) \quad \cdots (18)$$

In the above expression, $p_{t,l}^{doi}(t)$ is a first light emission probability model considering the uncertainty of the light emission position in the scintillator. Also, $p_{ph,l}^{doi}(t)$ is a light emission distribution function model considering the uncertainty of the light emission position at the scintillator position.

By explicitly expressing the parameters $\theta_l$ obtained by the processing circuitry 150 while employing the obtaining function 150a at step S100 as arguments of a mathematical function, it is also possible to rewrite expression (18) as expressions (19) and (20) presented below.

$$\theta_l = \left(N_{c;l}, N_{s;l}, \sigma_{c;l}, \sigma_{s;l} \ ...\right) \quad \cdots (19)$$

$$p_{t;l}^{doi}(\theta_l, t) = (N_{s;l} + N_{c;l})p_{ph;l}^{doi}(\theta_l, t) \exp\left(-(N_{s;l} + N_{c;l})\int_0^t p_{ph;l}^{doi}(\theta_l, t')\,dt'\right) \quad \cdots (20)$$

In the above expressions, $\theta_l$ denotes the parameter set obtained at the detector l, e.g., a set of parameters such as the scintillation photon number $N_{s;l}$; the Cherenkov photon number $N_{c;l}$; the time resolution $\sigma_c$ of the optical sensor regarding the Cherenkov light; and the time resolution $\sigma_s$ of the optical sensor regarding the scintillation light.

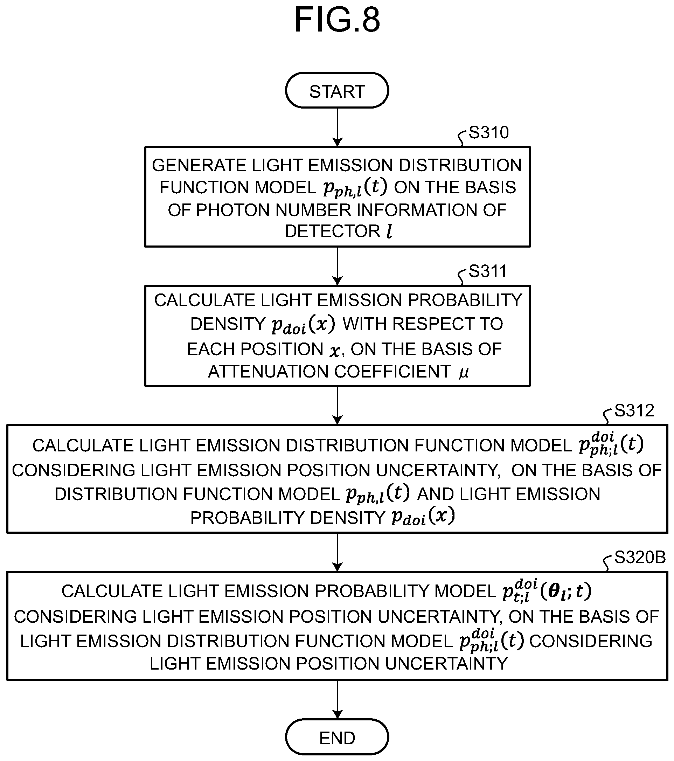

Returning to the explanation of Expression (18), the process at step S300 in the situation considering the uncertainty of the light emission position at the scintillator position will be explained. FIG. 8 is a flowchart illustrating a flow in the light emission probability model calculation considering the uncertainty of the light emission position in the scintillator.

Similarly to the example in FIG. 4, at step S310, by employing the calculating function 150b, the processing circuitry 150 generates the light emission distribution function model $p_{ph,l}(t)$, on the basis of the parameters $\theta_l$ such as the first photon number information of the first detector l and the like.

Subsequently, at step S311, by employing the calculating function 150b, the processing circuitry 150 calculates, with respect to each position x in the scintillator, light emission probability density $p^{doi}(x)$ on the basis of the value of an attenuation coefficient $\mu$. It is possible to express the light emission probability density $p^{doi}(x)$ at the position x by using Expression (21) presented below.

$$p_{doi}(x) = \frac{\mu \exp(-\mu x)}{1 - \exp(-\mu L)} \quad \cdots (21)$$

In this situation, the light emission probability density $p^{doi}(x)$ is standardized as indicated in Expression (22) presented below.

$$\int_0^L p_{doi}(x)dx = 1 \quad \cdots (22)$$

Further, in FIG. 7, a graph 45 expresses the attenuation ratio $\mu\exp(-\mu x)$.

Returning to the description of FIG. 8, at step S312, by employing the calculating function 150b, the processing circuitry 150 calculates the light emission distribution function model $p_{ph;l}^{doi}(t)$ considering the light emission position uncertainty, on the basis of the distribution function model $p_{ph,l}(t)$ generated at step S310 and the light emission probability density $p^{doi}(x)$.

More specifically, by employing the calculating function 150b, the processing circuitry 150 calculates the light emission distribution function model $p_{ph;l}^{doi}(t)$ considering the light emission position uncertainty, on the basis of the distribution function model $p_{ph,l}(t)$ generated at step S310 and the light emission probability density $p^{doi}(x)$ calculated at step S311, by using Expression (23) presented below.

$$p_{ph}^{doi}(t) = \int_0^L p_{doi}(x)p_{ph}(t - t')dx \quad \cdots (23)$$

In the above expression, L denotes the length of the scintillator; x denotes the light emission position; and t' denotes, as explained with Expression (17), delay time from when the gamma ray becomes incident to the scintillator, to when the scintillation light reaches the end of the scintillator.

After that, at step S320B, by employing the calculating function 150b, the processing circuitry 150 calculates a light emission probability model $p_{t;l}^{doi}(\theta_{l;t})$ considering the light emission position uncertainty, on the basis of the light emission distribution function model $p_{ph;l}^{doi}(t)$ considering the light emission position uncertainty. More specifically, as indicated in Expressions (24) and (25), the processing circuitry 150 calculates, by employing the calculating function 150b, the light emission probability model $p^{doi}(t)$ considering the light emission position uncertainty, on the basis of the light emission distribution function model $p_{ph}^{doi}(t)$ considering the light emission position uncertainty, similarly to the example using Expression (13).

$$p_t^{doi}(t) = N p_{ph}^{doi}(t) \exp\left(-N F^{doi}(t)\right) \quad \cdots (24)$$

$$F_{doi}(t) = \int_0^t p_{ph}^{doi}(t')dt' \quad \cdots (25)$$

In this situation, when a change of variables is applied to Expression (23) on the basis of Expression (18), Expression (26) presented below is obtained. Accordingly, by employing the calculating function 150b, the processing circuitry 150 may perform calculation by using Expression (26) when evaluating the right-hand side of Expression (24).

$$p_{ph}^{doi}(t) = \frac{c}{n - 1} \int_{\frac{L}{c}}^{\frac{nL}{c}} p_{doi}\left(\frac{nL - ct'}{n - 1}\right) p_{ph}(t - t')dt' \quad \cdots (26)$$

In the manner described above, by employing the calculating function 150b, the processing circuitry 150 is configured to calculate the first light emission probability model and the second light emission probability model, while taking the uncertainty of the light emission position in the scintillator into consideration.

Returning to the description of FIG. 2, at step S400, by employing the calculating function 150b, the processing circuitry 150 calculates a second light emission probability model $P_{t;m}^{(doi)}$ corresponding to the second detector m, on the basis of the second photon number information obtained at step S200, by performing a process similar to that at step S300.

More specifically, by employing the calculating function 150b, the processing circuitry 150 is configured to generate a light emission distribution function model $p_{ph;m}(t)$ by using expressions corresponding to Expressions (1), (4), (5), and (6), or the like, for example, on the basis of the second photon number information of the second detector m obtained at step S200.

Subsequently, by employing the calculating function 150b, the processing circuitry 150 is configured to calculate a second light emission probability model $p_{t;m}(t)$ by using expressions corresponding to Expressions (13) and (14) or the like, on the basis of the second photon number information and the light emission distribution function model $p_{ph;m}(t)$ of the second detector m.

Further, the second light emission probability model $p_{t,m}(t)$ is based on the expected photon number values $N_{s;m}$ and $N_{c;m}$ corresponding to the detection event.

Further, when calculating the second light emission probability model, the processing circuitry 150 is also capable, by employing the calculating function 150b, of performing a calculation considering the uncertainty of the light emission position in the scintillator, by using expressions similar to Expressions (17) and (21) to (26). In that situation, by employing the calculating function 150b, the processing circuitry 150 is configured to calculate the light emission probability density $p^{doi}(x)$ with respect to each position x in the scintillator, on the basis of the value of the attenuation coefficient $\mu$. Subsequently, by employing the calculating function 150b, the processing circuitry 150 is configured to calculate a light emission distribution function model $p_{ph;m}^{doi}(t)$ considering the light emission position uncertainty, on the basis of the distribution function model $p_{ph;m}(t)$ and the light emission probability density $p^{doi}(x)$. After that, by employing the calculating function 150b, the processing circuitry 150 is configured to calculate a light emission probability model $p_{t;m}^{doi}(t)$ considering the light emission position uncertainty, on the basis of the light emission distribution function model $p_{ph;m}^{doi}(t)$ considering the light emission position uncertainty.

In the manner described above, by employing the calculating function 150b, the processing circuitry 150 is configured to calculate the second light emission probability model $p_{t,m}(t)$, by taking the uncertainty of the light emission position in the scintillator into consideration.

After that, returning to the description of FIG. 2, at step S500, the processing circuitry 150 specifies, by employing the specifying function 150c, a probability distribution model $p_{TOF;l,m}^{doi}(\theta_l,\theta_m,t)$ related to a detection time difference along a LOR defined by the first detector l and the second detector m, on the basis of a first light emission probability model $p_{t;l}^{doi}(t_1)$ and a second light emission probability model $p_{t;m}^{doi}(t_2)$.

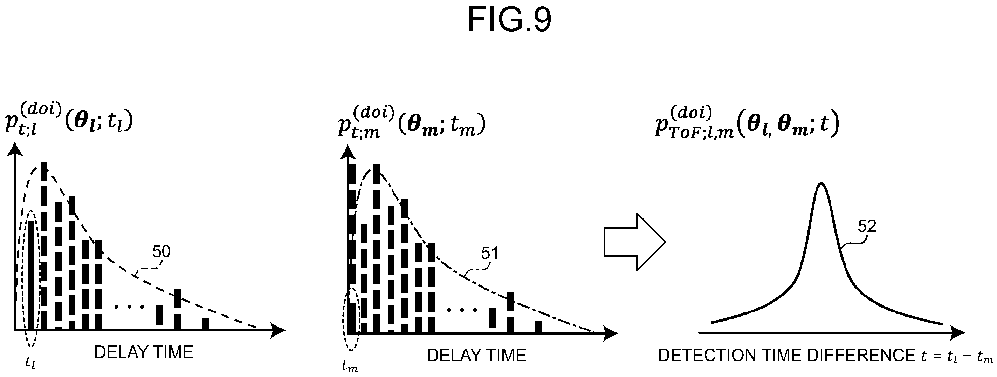

In FIG. 9, a curve 50 expresses a result of plotting the first light emission probability model $p_{t;l}^{doi}(\theta_l, t_l$ of the first detector l as a mathematical function of delay time $t_l$. Also, another curve 51 expresses a result of plotting the second light emission probability model $p_{t;m}^{doi}(\theta_m,t_m)$ of the second detector m as a mathematical function of delay time $t_m$. By employing the specifying function 150c, the processing circuitry 150 is configured to specify the probability distribution model $p_{TOF;l,m}^{do}(\theta_l,\theta_m,t)$ which expresses the event in the detector l at the detection time $t_1$ and an intersection of events in the detector m at the detection time tm, as a mathematical function of the detection time difference. Yet another curve 52 expresses an example of the probability distribution model $p_{TOF;l,m}^{doi}(\theta_l,\theta_m,t)$ specified in this manner.

In this situation, for the duration of a time width dt, it is possible to express the number of the events in the detector l at the detection time $t_1$ and the number of the intersections of events in the detector m at the detection time $t_m$, by using Expression (27) presented below.

$$p_{t;l}^{(doi)}(t_l)p_{t;m}^{(doi)}(t_m)dt \qquad \cdots (27)$$

Further, it is possible to express the detection time difference between the detection time $t_l$ of the detector l and the detection time $t_m$ of the detector m, by using Expression (28) presented below.

$$t = t_l - t_m \qquad \cdots (28)$$

Consequently, it is possible to express $p_{TOF;l,m}^{doi}(\theta_l,\theta_m,t)$ by using Expression (29) presented below.

$$p_{ToF;l,m}^{(doi)}(t) = \int_0^\infty p_{t;l}^{(doi)}(t')p_{t;m}^{(doi)}(t' - t)dt' \qquad \cdots (29)$$

As explained above, by employing the calculating function 150b, the processing circuitry 150 is configured to calculate the probability distribution model $p_{TOF;l,m}^{doi}(\theta_l,\theta_m,t)$ related to the detection time difference along the LOR defined by the first detector l and the second detector m on the basis of Expression (29). By using the probability distribution model as a ToF kernel in the PET image reconstruction, the processing circuitry 150 is able to have the difference in the ToF resolutions among the detectors reflected on the PET image reconstruction and is thus able to enhance image quality of reconstructed images.

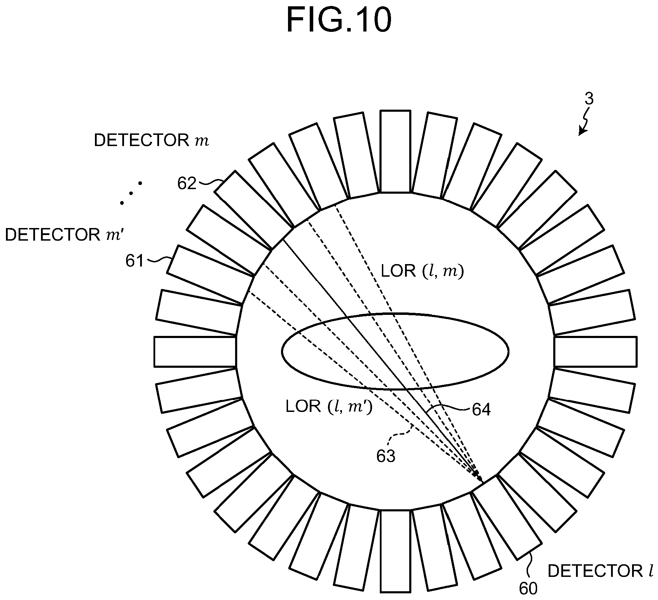

For example, as illustrated in FIG. 10, by employing the calculating function 150b, the processing circuitry 150 is configured to calculate, with respect to each of the LORs, the probability distribution model $p_{TOF;l,m}{}^{doi}(\theta_l,\theta_m,t)$ related to the detection time difference along the LOR. For example, by employing the calculating function 150b, the processing circuitry 150 is configured to calculate a probability distribution model $p_{TOF;l,m'}{}^{doi}(\theta_l,\theta_{m'},t)$ related to an LOR 63 between a detector 60 and another detector 61 and to also calculate the probability distribution model $p_{TOF;l,m}{}^{doi}(\theta_l,\theta m,t)$ related to an LOR 64 between the detector 60 and yet another detector 62. With this configuration, it is possible to generate an individual probability distribution model for each of the detectors. Consequently, even when there is variance in the capabilities among the detectors, for example, it is possible to enhance image quality of the reconstructed images.

Returning to the description of FIG. 2, at step S600, by employing the reconstructing function 150d, the processing circuitry 150 generates a reconstructed image by reconstructing the data corresponding to the LOR, by using the probability distribution model $p_{TOF;l,m}{}^{doi}(\theta_l,\theta_m,t)$ specified at step S500, as a ToF kernel. In other words, by employing the reconstructing function 150d, the processing circuitry 150 is configured to reconstruct the data corresponding to the LOR, by using the probability distribution model related to the detection time difference as the Time Of Flight (ToF) kernel.

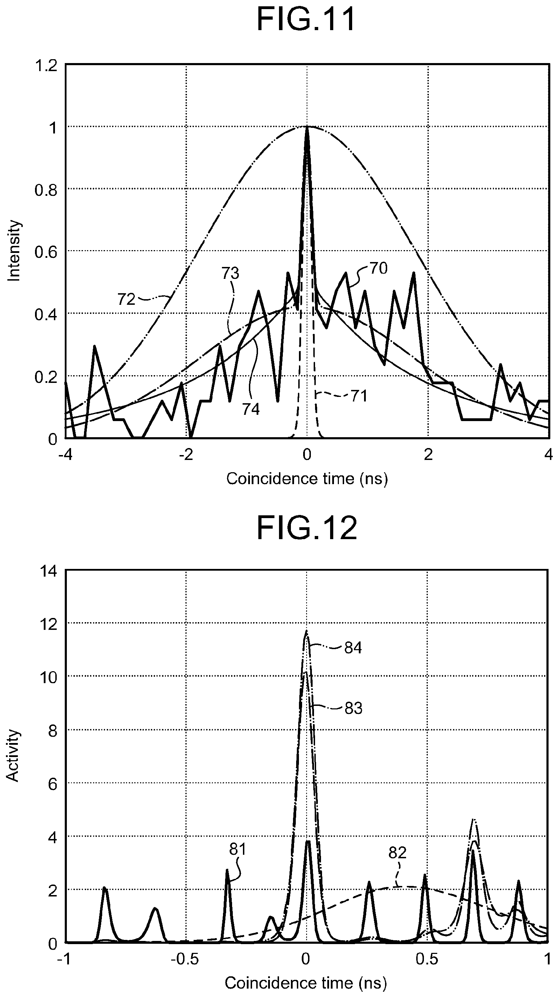

FIGS. 11 and 12 illustrates a result of verifying the method according to the first embodiment. In this verification, capabilities of the ToF kernel generated by using the method according to the first embodiment were evaluated by using a 1D Maximum Likelihood Expectation Maximization (1D-ML-EM) method.

In the situation where simultaneous measuring of a radioactive body in a position j occurs in a position i, when spatial coordinates are defined with coincidence times, it is possible to express a coincidence spectrum y(i) by using Expression (30) presented below, where $c_{ji}$ denotes the ToF kernel, whereas λ(j) denotes a distribution of a radioactive substance.

$$y[i] = \sum_j c_{ji}\lambda[j] \quad \cdots (30)$$

In this situation, when a distribution of the radioactive substance is calculated by using the ML-EM method, it is possible to express a distribution $\lambda^{k+1}$ of the radioactive substance at a (k+1)-th update, by using Expression (31) presented below.

$$\lambda^{k+1}[j] = \frac{\lambda^k[j]}{\sum_i c_{ji}} \sum_i c_{ji} \frac{y[i]}{\sum_{j'} c_{j'i}\lambda^k[j']} \quad \cdots (31)$$

In this situation, it is considered that, if the ToF kernel is valid, the distribution of the radioactive substance reconstructed by using the ML-EM method will have a peak in a narrow region. Thus, we reconstructed the distribution of the radioactive substance, by using various ToF kernels.

More specifically, we used four types of ToF kernels as the different types of ToF kernels to be studied. In other words, we studied the following four kernels: a plurality of Gaussian kernels using a sum of a Gaussian distribution of Cherenkov light and a Gaussian distribution of scintillation light; a single Gaussian kernel postulating only scintillation light; a single Gaussian kernel postulating only Cherenkov light; and a model-based kernel considering both Cherenkov light and scintillation light by using a model-based scheme of the light emission model according to the first embodiment. As illustrated in FIG. 11, results were fitted against an actual measured spectrum 70. Curves 73, 72, 71, and 74 indicate results of the fitting using the plurality of Gaussian kernels, the single Gaussian kernel postulating only scintillation light, the single Gaussian kernel postulating only Cherenkov light, and the model-based kernel, respectively.

FIG. 12 illustrates a result of reconstructing the distribution of the radioactive substance, by performing the reconstructions with the ML-EM method, while using the four types of ToF kernels. In FIG. 12, curves 83, 82, 81, and 84 indicate calculation results from the reconstructions using the plurality of Gaussian kernels, the single Gaussian kernel postulating only scintillation light, the single Gaussian kernel postulating only Cherenkov light, and the model-based kernel, respectively. When the reconstruction was performed by using the model-based kernel, the width between the peaks of the radioactive substance reconstructed by implementing the ML-EM method was narrow. Thus, we were able to confirm validity of the ToF kernel generated by using the method according to the first embodiment.

As explained above, in the nuclear medicine diagnosis apparatus according to the first embodiment, the processing circuitry 150 is configured to calculate, with respect to each of the detectors, the probability distribution model related to the detection time difference, on the basis of the light emission model in the scintillator. Accordingly, it is possible to perform the image reconstruction by setting the individual ToF kernel for each of the LORs. Consequently, even when there is variance in the capabilities among the detectors, for example, it is possible to enhance image quality of the PET images resulting from the reconstruction.

Possible embodiments are not limited to the examples described above. Although the examples were explained above in which the detectors 3 are ordinary detectors and do not structure a monolithic detector, possible embodiments are not limited to those examples. The embodiments are also applicable to the situation where a detector 3 is a monolithic scintillator structured as a single crystal, so that the single scintillator crystal is used in an integral form without being divided. The scintillator is formed by using the scintillator crystal of Lutetium Yttrium Oxyorthosilicate (LYSO), Lutetium Oxyorthosilicate (LSO), Lutetium Gadolinium Oxyorthosilicate (LGSO), or the like, for example.

When the detector 3 is a monolithic detector, the detector 3 is configured so that the single scintillator crystal is used in the integral form without being divided; however, on the basis of the concept where the detector 3 is structured with a plurality of virtual detectors, it is possible to perform the same processes by generating a ToF kernel corresponding to each set of virtual detectors. For example, when the detector 3 includes a plurality of detecting element arrays, it is possible to perform the same processes as those in the flowchart of FIG. 2, by considering each of the detecting element arrays as one detector. In an example, by performing the same processes while replacing the numbers assigned to the detectors with numbers assigned to the detecting element arrays, regarding the natural numbers l and m used at steps S100 through S600 in FIG. 2 and in Expressions (20), (29), and so on, the processing circuitry 150 is able, by the specifying function, to specify a probability distribution model related to a detection time difference corresponding to the set of detecting element arrays.

In other words, when the detector 3 is a monolithic detector, a nuclear medicine diagnosis apparatus according to an embodiment includes: the detector 3 structured with the monolithic detector including at least a first detecting element array l and a second detecting element array m; and the processing circuitry 150. By employing the obtaining function 150a, the processing circuitry 150 is configured to obtain first photon number information detected by the first detecting element array l and second photon number information detected by the second detecting element array m. By employing the calculating function 150b, the processing circuitry 150 is configured to calculate a first light emission probability model $p_{t;l}^{doi}(\theta_l,t_l)$ corresponding to the first detecting element array l on the basis of the first photon number information and to calculate a second light emission probability model $p_{t;m}^{doi}(\theta_m,t_m)$ corresponding to the second detecting element array m on the basis of the second photon number information. By employing the specifying function 150c, the processing circuitry 150 is configured to specify a probability distribution model $p_{TOF;l,m}^{doi}(\theta_l,\theta_m,t)$ related to a detection time difference corresponding to the set made up of the first detecting element array l and the second detecting element array m, on the basis of the first light emission probability model $p_{t;l}^{doi}(\theta_l,t_l)$ and the second light emission probability model $p_{t;m}^{doi}(\theta_m,t_m)$.

Further, a data processing method according to an embodiment includes: obtaining, with respect to a monolithic detector including at least the first detecting element array l and the second detecting element array m, first photon number information detected by the first detecting element array l and second photon number information detected by the second detecting element array m; calculating a first light emission probability model $p_{t;l}^{doi}(\theta_l,t_l)$ corresponding to the first detecting element array l on the basis of the first photon number information and calculating a second light emission probability model $p_{t;m}^{doi}(\theta_m,t_m)$ corresponding to the second detecting element array m on the basis of the second photon number information; and specifying a probability distribution model $p_{TOF;l,m}^{doi}(\theta_l,\theta_m,t)$ related to a detection time difference corresponding to the set made up of the first detecting element array l and the second detecting element array m, on the basis of the first light emission probability model $p_{t;l}^{doi}(\theta_l,t_l)$ and the second light emission probability model $p_{t;m}^{doi}(\theta_m,t_m)$.

Further, a program according to an embodiment causes a computer to execute: obtaining, with respect to a monolithic detector including at least the first detecting element array l and the second detecting element array m, first photon number information detected by the first detecting element array l and second photon number information detected by the second detecting element array m; calculating a first light emission probability model $p_{t;l}^{doi}(\theta_l,t_l)$ corresponding to the first detecting element array l on the basis of the first photon number information and calculating a second light emission probability model $p_{t;m}^{doi}(\theta_m,t_m)$ corresponding to the second detecting element array m on the basis of the second photon number information; and specifying a probability distribution model $p_{TOF;l,m}^{doi}(\theta_l,\theta_m,t)$ related to a detection time difference corresponding to the set made up of the first detecting element array l and the second detecting element array m, on the basis of the first light emission probability model $p_{t;l}^{doi}(\theta_l,t_l)$ and the second light emission probability model $p_{t;m}^{doi}(\theta_m,t_m)$.

Second Embodiment

To begin with, a background of a second embodiment will be explained. To reconstruct a PET image, the image reconstruction is performed by using a Time Of Flight (ToF) kernel. The ToF kernel denotes a probability density function of a detection event expressed as a mathematical function of a detection time difference between signals detected by two detectors. In this situation, when the time width of the ToF kernel used in the image reconstruction does not match the ToF capabilities of the detectors, the image quality of a reconstructed image may be degraded. For example, when the time width of the ToF kernel is smaller than the ToF capabilities of the detectors, estimated positions indicating radioactive substance positions after the image reconstructions may be more concentrated locally than those in an actual distribution.

On the contrary, when the time width of the ToF kernel is larger than the ToF capabilities of the detectors, estimated positions indicating radioactive substance positions after the image reconstructions may be distributed more in peripheral parts than those in the actual distribution. Accordingly, it is desirable to have the ToF capabilities of the detectors match the ToF kernel used for the image reconstruction as much as possible.

However, when there are a small number of events, for example, variation in the measurement will be large, and there may be an error in the calculation of the ToF kernels. To cope with this situation, in the second embodiment, a probability distribution model is generated for each coincidence event on the basis of a light emission model, and a ToF kernel is calculated by adding up the probability distributions. Accordingly, it is possible to perform the image reconstruction by generating the ToF kernel that takes into consideration the variation of each event from an average of measurement values. Consequently, even when there are a small number of coincidence events that make the variation in the measurement seem large, for example, it is possible to enhance image quality of the PET image resulting from the reconstruction.

On the basis of the background described above, a nuclear medicine diagnosis apparatus according to the second embodiment includes the processing circuitry 150. By employing the obtaining function 150a, the processing circuitry 150 is configured to obtain first photon number information related to a first event detected by a first detector and second photon number information related to a second event detected by a second detector different from the first detector. By employing the specifying function 150c, the processing circuitry 150 is configured to specify a probability distribution model related to coincidence defined based on the first event and the second event, on the basis of the first photon number information and the second photon number information.

Further, a data processing method according to the second embodiment includes: measuring first photon number information related to a first event detected by a first detector and second photon number information related to a second event detected by a second detector different from the first detector; and specifying a probability distribution model related to coincidence defined based on the first event and the second event, on the basis of the first photon number information and the second photon number information.

Further, a program according to the second embodiment causes a computer to execute: measuring first photon number information related to a first event detected by a first detector and second photon number information related to a second event detected by a second detector different from the first detector; and specifying a probability distribution model related to coincidence defined based on the first event and the second event, on the basis of the first photon number information and the second photon number information.

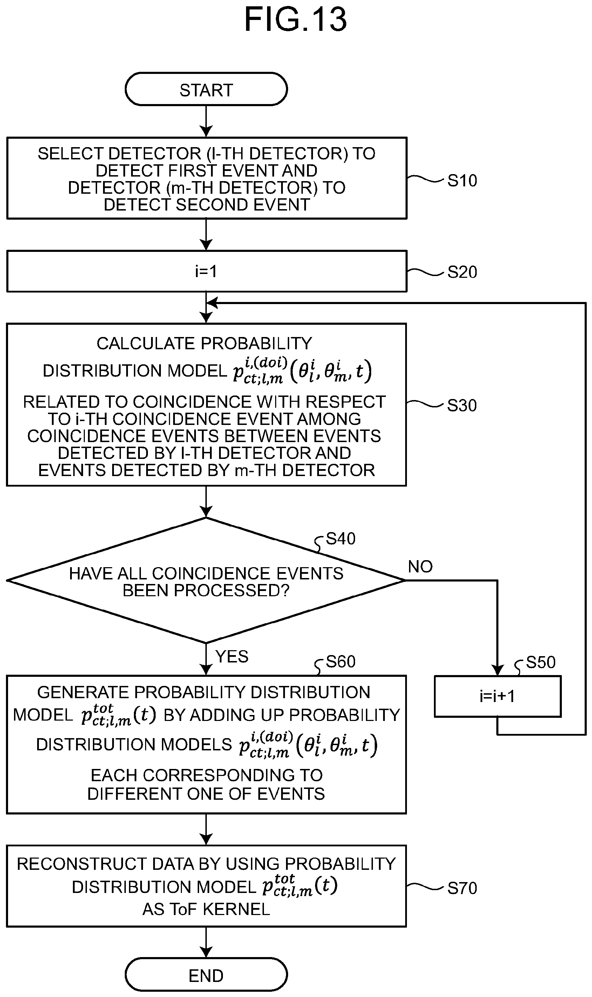

Next, processes performed by a nuclear medicine diagnosis apparatus according to the second embodiment will be explained in detail below, with reference to FIGS. 13 to 17.

FIG. 13 illustrates a flow of processes performed by the PET apparatus 100 serving as the nuclear medicine diagnosis apparatus according to the second embodiment. To simplify the explanations, the following flowchart will only describe the situation where a probability distribution model (a ToF kernel) is generated for the first detector l and the second detector m. However, in image reconstruction, the processing circuitry 150 is typically configured to perform the image reconstructing process at step S70 after generating a probability distribution model with respect to every set of l and m. In that situation, the processing circuitry 150 is configured to perform the process at step S70 after repeatedly performing the processes at steps S10 through S60 with respect to every set of l and m.

At step S10, the processing circuitry 150 selects values of l and m indicating a set of detectors to be processed. In other words, the processing circuitry 150 selects the values of l and m characterizing a first detector l (an l-th detector) which is a detector to detect a first event and a second detector m (an m-th detector) which is a detector to detect a second event. In this situation, the first event and the second event structure one coincidence event. In other words, each of coincidence events is structured with two events, namely a first event and a second event.

Subsequently, at step S20, the processing circuitry 150 sets the value of i indicating a coincidence event number to 1. In this situation, "i = 1" denotes that the processing circuitry 150 is to process the first coincidence event.

Subsequently, at step S30, by employing the specifying function 150c, the processing circuitry 150 calculates a probability distribution model $p_{ct;l,m}^{i,(doi)}(\theta_l^i, \theta_m^i, t)$ related to coincidence, with respect to an i-th coincidence event among the coincidence events between events detected by the l-th detector and events detected by the m-th detector. In this situation, $\theta_l^i$ and $\theta_m^i$ denote parameters obtained with respect to the l-th detector and the m-th detector, respectively, in the i-th coincidence event. Further, the symbol (doi) indicates that the uncertainty of the light emission position in the scintillator may be taken into consideration or may not be taken into consideration. It means that, by employing the specifying function 150c, the processing circuitry 150 is configured to calculate a probability distribution model $p_{ct;l,m}^{i,doi}(\theta_l^i, \theta_m^i, t)$ related to coincidence considering the uncertainty of the light emission position in the scintillator in the embodiment considering the uncertainty of the light emission position in the scintillator and is configured to calculate a probability distribution model $p_{ct;l,m}^i(\theta_l^i, \theta_m^i, t)$ related to coincidence not considering the uncertainty of the light emission position in the scintillator in the embodiment not considering the uncertainty of the light emission position in the scintillator.

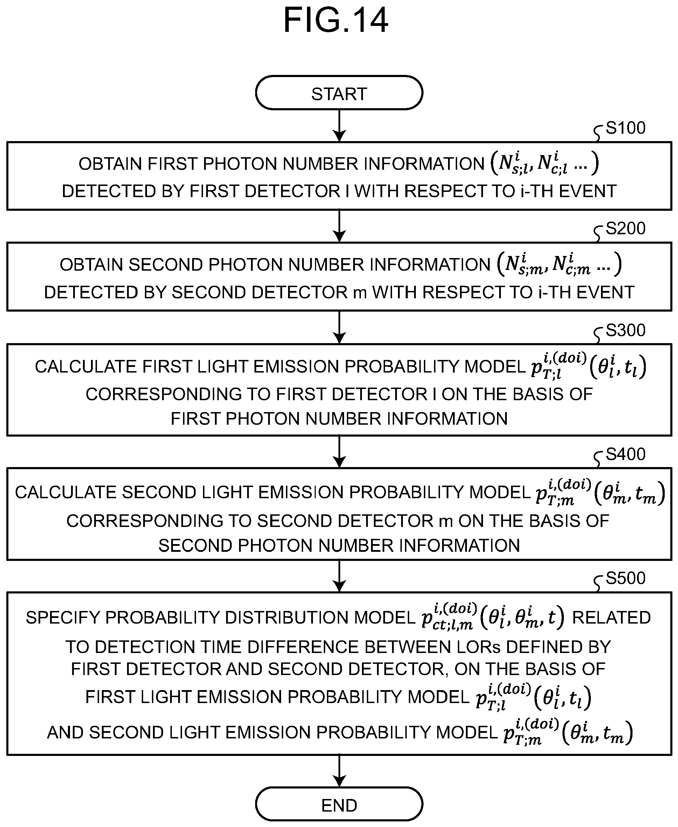

FIG. 14 illustrates a flow in the process further in detail, regarding the process at step S30 in FIG. 13. In other words, steps S100 through S500 in FIG. 14 explain step S30 in FIG. 13 further in detail.

To begin with, at step S100, by employing the obtaining function 150a, the processing circuitry 150 obtains first photon number information related to the first event detected by the first detector l, with respect to the i-th coincidence event.

In this situation, the first photon number information may be, for example, a scintillation photon number $N^i_{s;l}$ with respect to the event in question. In another example, the first photon number information may be a Cherenkov photon number $N^i_{c;l}$ with respect to the event in question, for instance. In yet another example, the first photon number information may be the scintillation photon number $N^i_{s;l}$ and the Cherenkov photon number $N^i_{c;l}$ with respect to the event in question. In other words, by employing the obtaining function 150a, the processing circuitry 150 is configured to obtain the scintillation photon number $N^i_{s;l}$, the Cherenkov photon number $N^i_{c;l}$, or the scintillation photon number $N^i_{s;l}$ and the Cherenkov photon number $N^i_{c;l}$, as the first photon number information. In this situation, the first photon number information obtained by the processing circuitry 150 while employing the obtaining function 150a is obtained with respect to each event.

In this situation, the PET apparatus 100 may obtain the first photon number information on the basis of an energy spectrum obtained by the first detector l.

In this situation, as previously explained in the first embodiment, it is possible to express the photon density $n_c(t)$ of the Cherenkov light by using Expression (1) where $N_c$ denotes the photon number of the Cherenkov light, $\sigma_c$ denotes the half width of the Cherenkov light spectrum, and $t_0$ denotes the peak time of the Cherenkov light. Further, it is possible to express the excitation density $n_{ex}(t)$ of the valence electron by using Expression (2), where $N_s$ denotes the photon number of the scintillation light, $\sigma_s$ denotes the half width of the scintillation light spectrum, and $t_1$ denotes the peak time of the scintillation light. Furthermore, it is possible to express the photon density $n_s(t)$ of the scintillation light by using Expression (3) which uses the excitation density $n_{ex}(t)$ of the valence electron and in which $\tau_s$ denotes the relaxation constant. Consequently, when a function form of the excitation density $n_{ex}(t)$ of the valence electron is assumed to be Expression (2), substituting Expression (2) into Expression (3) gives Expression (4).

In other words, the PET apparatus 100 may be configured: to obtain the energy spectra obtained the first detector l and the second detector m; to generate the spectra expressed by the curve 17 and the curve 22 on the basis of the energy spectra; and to obtain the first photon number information and the second photon number information on the basis of the generated spectra.

In this situation, because the light emission distribution function $p_{ph}(t)$ is in proportion to the sum of the photon density $n_c(t)$ of the Cherenkov light and the photon density $n_s(t)$ of the scintillation light, Expression (5) holds true.

In this situation, the light emission distribution function $p_{ph}(t)$ is standardized as indicated in Expression (6).

Further, at step S100, the processing circuitry 150 may obtain information other than the photon number information as a parameter set related to the first detector l, by employing the obtaining function 150a. In an example, the processing circuitry 150 may be configured, by employing the obtaining function 150a, to obtain a time resolution $\sigma_c$ of the optical sensor regarding the Cherenkov light, in place of the first photon number information or in addition to the first photon number information, as one of parameter sets $\theta_l$ obtained by the first detector l. In another example, the processing circuitry 150 may be configured, by employing the obtaining function 150a, to obtain a time resolution $\sigma_s$ of the optical sensor regarding the scintillation light, in place of the first photon number information or in addition to the first photon number information, as one of the parameter sets $\theta_l$ obtained by the first detector l. In yet another example, the processing circuitry 150 may be configured, by employing the obtaining function 150a, to obtain an excessive noise occurrence probability, as one of the parameter sets $\theta_l$ obtained by the first detector l. In this situation, the excessive noise occurrence probability denotes, for example, an occurrence probability of Avalanche Photodiode (APD) excessive noise such as optical crosstalk or afterpulsing, for example.

At step S200, by employing the obtaining function 150a, the processing circuitry 150 obtains second photon number information related to the second event detected by the second detector m different from the first detector l, with respect to the i-th coincidence event.

In this situation, the second photon number information may be, for example, a scintillation photon number $N^i_{s;m}$ with respect to the coincidence event in question. In another example, the second photon number information may be a Cherenkov photon number $N^i_{c;m}$ with respect to the coincidence event in question, for instance. In yet another example, the second photon number information may be the scintillation photon number $N^i_{s;m}$ and the scintillation photon number $N^i_{s;m}$ with respect to the coincidence event in question. In other words, by employing the obtaining function 150a, the processing circuitry 150 is configured to obtain the scintillation photon number $N^i_{s;m}$, the Cherenkov photon number $N^i_{c;m}$, or the scintillation photon number $N^i_{s;m}$ and the Cherenkov photon number $N^i_{c;m}$, as the second photon number information. In this situation, the second photon number information obtained by the processing circuitry 150 while employing the obtaining function 150a is obtained with respect to each event.

The PET apparatus 100 may obtain the second photon number information on the basis of an energy spectrum obtained by the second detector m.

Further, the processing circuitry 150 may be configured, by employing the obtaining function 150a, to obtain the time resolution $\sigma_c$ of the optical sensor regarding the Cherenkov light, the time resolution $\sigma_s$ of the optical sensor regarding the scintillation light, and the excessive noise occurrence probability, or the like, as the parameter set $\theta$ obtained by the

second detector m.

At step S300, by employing the calculating function 150b, the processing circuitry 150 calculates a first light emission probability model $p_{t;l}^{i,(doi)}$ corresponding to the first detector l in the i-th coincidence event, on the basis of the first photon number information obtained at step S100. In this situation, by employing the calculating function 150b, the processing circuitry 150 is configured to determine the first light emission probability model $P_{t;l}^{i,(doi)}$ for the first detector l, with respect to each coincidence event.

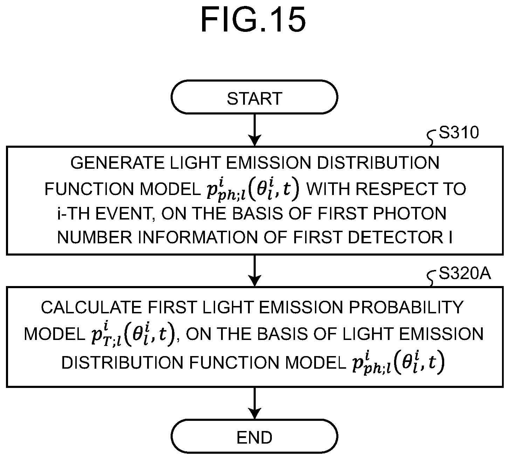

FIG. 15 illustrates an example of the process at step S300 further in detail. The flowchart in FIG. 15 is a flowchart explaining, further in detail, the example of the process at step S300.

At step S310, by employing the calculating function 150b, the processing circuitry 150 generates a light emission distribution function model $p_{ph,l}^{i}(t)$ on the basis of the first photon number information of the first detector l obtained at step S100. The light emission distribution function $p_{ph}(t)$ is a distribution function indicating a probability that light emission may occur at the time t. When the light emission distribution function model $p_{ph,l}^{i}(t)$ is integrated with respect to times over the total period, the result is 1. Also, the light emission distribution function model $p_{ph,l}^{i}(t)$ is determined with respect to each of the detectors and with respect to each coincidence event.

By employing the calculating function 150b, the processing circuitry 150 is configured to generate a light emission distribution function model $p_{ph,l}^{i}(t)$ by using Expression (1), (4), (5), and (6) or the like, on the basis of the first photon number information, as previously explained in the first embodiment, for example. Because the first photon number information is obtained with respect to each of the detectors and with respect to each coincidence event, the processing circuitry 150 is configured, by employing the calculating function 150b, to calculate a light emission distribution function model $p_{ph,l}^{i}(t)$ with respect to each of the detectors and with respect to each coincidence event. As previously explained in the first embodiment, FIG. 5 illustrates an example of the light emission distribution function $p_{ph}(t)$. In FIG. 5, the curve 30 expresses the light emission distribution function $p_{ph}(t)$. Further, the threshold values (N') of the photons for defining the detection times are also indicated.

Subsequently, at step S320A, by employing the calculating function 150b, the processing circuitry 150 calculates a first light emission probability model $p_{t,l}^{i}(\theta_{l}^{i},t)$ on the basis of the light emission distribution function model $p_{ph,l}^{i}(\theta_{l}^{i},t)$.

In this situation, the first light emission probability model $p_{t,l}^{i}(\geq N',\theta_{l}^{i},t)$ is a model related to probability density for detecting the first N' or more photons among the plurality of photons included in the detection event. In other words, the first light emission probability model $p_{t,l}(\geq N',t)$ is a model related to the probability density for detecting the prescribed number of photons among the plurality of photons included in the detection event.

As previously explained in the first embodiment, Expressions (7) to (13) hold true.

In other words, by employing the calculating function 150b, the processing circuitry 150 is able to calculate the first light emission probability model pt(t), on the basis of the light emission distribution function $p_{ph}(t)$, by using Expressions (12) and (13). In this manner, by employing the calculating function 150b, the processing circuitry 150 is configured to calculate the first light emission probability model pt(t) with respect to the first event, on the basis of the first photon number information and the light emission distribution function model $p_{sh}(t)$ of the first detector determined for each coincidence event, by substituting the light emission distribution function model $p_{ph}(t)$ of the first detector into Expressions (12) and (13) and further substituting the N on the right-hand side of Expression (13) with the first photon number information and evaluating the right-hand side of Expression (13).

In this situation, it is possible to determine the light emission distribution function $p_{ph}(t)$ and the first light emission probability model pt(t) described above, with respect to each of the detectors and each coincidence event. Accordingly, by explicitly indicating the subscripts of the detectors and the subscripts of the coincidence events, it is also possible to rewrite Expression (13) as Expression (32) presented below.

$$p_{t;l}^{i}(t) = (N_{s;l}^{i} + N_{c;l}^{i})\, p_{ph;l}^{i}\big(N_{s;l}^{i}, N_{c;l}^{i}, t\big) \exp\left( -(N_{s;l}^{i} + N_{c;l}^{i}) \int_{0}^{t} p_{ph;l}^{i}\big(N_{s;l}^{i}, N_{c;l}^{i}, t'\big)\, dt' \right)$$

$$\cdots (32)$$

In the above expression, $p_{t;l}^{i}(t)$ denotes a first light emission probability model of the detector l with respect to the i-th coincidence event; $p_{ph;l}^{i}(t)$ denotes the light emission distribution function of the detector l with respect to the i-th coincidence event; $N_{s;l}^{i}$ denotes the photon number in the scintillation light at the detector l with respect to the i-th coincidence event; and $N_{c;l}^{i}$ denotes the photon number in the Cherenkov light at the detector l with respect to the i-th coincidence event.

Further, when calculating the first light emission probability model, the processing circuitry 150 is also capable, by employing the calculating function 150b, of performing the calculation considering the uncertainty of the light emission position in the scintillator.

This calculation was discussed in the first embodiment by using FIG. 8 and Expressions (15) to (18). As previously explained, Expressions (15) to (17) hold true.

Similarly to the first embodiment, to obtain a formula considering the light emission position x in the scintillator on the basis of Expression (13), when we address the notion of taking the light emission position x in the scintillator into consideration, Expression (33) presented below holds true.

$$p_{t;l}^{i,doi}(t) = (N_{s;l}^i + N_{c;l}^i)p_{ph;l}^{i,doi}(N_{s;l}^i, N_{c;l}^i, t) \exp\left(-(N_{s;l}^i + N_{c;l}^i)\int_0^t p_{ph;l}^{i,doi}(N_{s;l}^i, N_{c;l}^i, t')\, dt'\right)$$

$$\cdots (33)$$

In the above expression, $p_{t,l}^{i,doi}(t)$ is a first light emission probability model considering the uncertainty of the light emission position in the scintillator and being determined for each coincidence event. Further, $p_{ph,l}^{i,doi}(t)$ is a light emission distribution function model considering the uncertainty of the light emission position at the scintillator and being determined for each coincidence event.

By explicitly expressing the parameters $\theta_l$ obtained by the processing circuitry 150 while employing the obtaining function 150a at step S100 as arguments of a mathematical function, it is also possible to rewrite expression (33) as expressions (34) and (35) presented below.

$$\theta_l^i = \left(N_{c;l}^i, N_{s;l}^i, \sigma_{c;l}^i, \sigma_{s;l}^i \dots\right) \qquad \cdots (34)$$

$$p_{t;l}^{i,doi}\left(\theta_l^i, t\right) = (N_{s;l}^i + N_{c;l}^i)p_{ph;l}^{i,doi}\left(\theta_l^i, t\right) \exp\left(-(N_{s;l}^i + N_{c;l}^i)\int_0^t p_{ph;l}^{i,doi}\left(\theta_l^i, t'\right) dt'\right) \qquad \cdots (35)$$

In the above expressions, $\theta_l^i$ denotes a parameter set obtained at the detector l and being determined for each coincidence event and denotes, for example, a set of parameters such as a scintillation photon number $N_{s;l}^i$; a Cherenkov photon number $N_{c;l}^i$; a time resolution $\sigma_C^i$ of the optical sensor regarding the Cherenkov light; and a time resolution $\sigma_S^i$ of the optical sensor regarding the scintillation light that are determined for each coincidence event.

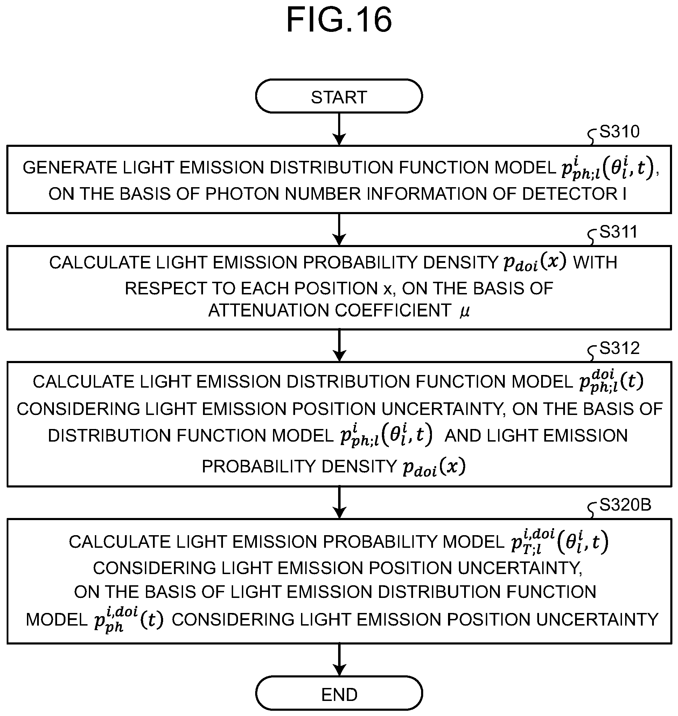

Returning to the explanation of Expression (33), a process at step S300 in the situation considering the uncertainty of the light emission position at the scintillator will be explained. FIG. 16 is a flowchart illustrating a flow in the light emission probability model calculation considering the uncertainty of the light emission position in the scintillator.

Similarly to the example in FIG. 15, at step S310, by employing the calculating function 150b, the processing circuitry 150 generates a light emission distribution function model $p_{ph,l}(t)$ with respect to each coincidence event, on the basis of the parameters $\theta_l$ such as the first photon number information of the first detector l and the like.

Subsequently, at step S311, by employing the calculating function 150b, the processing circuitry 150 calculates light emission probability density $p^{doi}(x)$ on the basis of the value of the attenuation coefficient $\mu$ with respect to each position x in the scintillator. It is possible to express the light emission probability density $p^{doi}(x)$ in the position x by using Expression (21). In this situation, the light emission probability density $p^{doi}(x)$ is standardized as indicated in Expression (22).

Returning to the description of FIG. 16, at step S312, by employing the specifying function 150c, the processing circuitry 150 calculates, as the first light emission probability model, the light emission distribution function model $p_{ph;l}^{i,doi}(t)$ considering the uncertainty of the light emission position and determined for each coincidence event, on the basis of the distribution function model $p_{ph,l}^i(t)$ generated at step S310, which is the light emission distribution function determined for each coincidence event, and the light emission probability density $p^{doi}(x)$. More specifically, by employing the specifying function 150c, the processing circuitry 150 calculates the light emission distribution function model $p_{ph;l}^{i,doi}(t)$ considering the light emission position uncertainty, on the basis of the distribution function model $p_{ph,l}^i(t)$ generated at step S310 and the light emission probability density $p^{doi}(x)$ calculated at step S311, by using Expression (23).

After that, at step S320B, by employing the specifying function 150c, the processing circuitry 150 calculates the light emission probability model $p_{t;l}^{i,doi}(\theta_{l;}t)$ considering the light emission position uncertainty and determined for each coincidence event, on the basis of the light emission distribution function model $p_{ph;l}^{i,doi}(t)$ considering the light emission position uncertainty and determined for each coincidence event. More specifically, as indicated in Expressions (24) and (25), the processing circuitry 150 calculates, by employing the specifying function 150c, the light emission probability model $p_t^{i,doi}(t)$ considering the light emission position uncertainty and determined for each coincidence event, on the basis of the light emission distribution function model $p_{ph}^{i,doi}(t)$ considering the light emission position uncertainty and determined for each coincidence event, similarly to the example in Expression (13).

In this situation, when the variable x in the integrand is changed to t' in Expression (23) on the basis of Expression (17), Expression (26) is obtained. Accordingly, by employing the specifying function 150c, the processing circuitry 150 may perform the calculation by using Expression (26) when evaluating the right-hand side of Expression (24).

As explained above, by employing the specifying function 150c, the processing circuitry 150 is configured to calculate the first light emission probability model and the second light emission probability model, while taking the uncertainty of the light emission position in the scintillator into consideration.

Returning to the description of FIG. 14, at step S400, by employing the specifying function 150c and performing a process similar to that at step S300, the processing circuitry 150 calculates a second light emission probability model $p^{i,(doi)}_{t;m}$ that corresponds to the second detector m and is determined for each coincidence event, on the basis of the second photon number information obtained at step S200.

More specifically, by employing the specifying function 150c, the processing circuitry 150 is configured to generate the light emission distribution function model $p^i_{ph;m}(t)$ determined for each coincidence event, by using expressions corresponding to Expressions (1), (4), (5), and (6), or the like, for example, on the basis of the second photon number information determined with respect to the second detector m for each coincidence event and obtained at step S200.

Subsequently, by employing the specifying function 150c, the processing circuitry 150 is configured to calculate a second light emission probability model $p^i_{t;m}(t)$ determined for each coincidence event by using an expression corresponding to Expression (14) or the like, on the basis of the second photon number information and the light emission distribution function model $p^i_{ph;m}(t)$ of the second detector m.

Further, when calculating the second light emission probability model, the processing circuitry 150 is also capable, by employing the calculating function 150b, of performing a calculation considering the uncertainty of the light emission position in the scintillator, by using expressions similar to Expressions (17) and (21) to (26). In that situation, by employing the calculating function 150b, the processing circuitry 150 is configured to calculate the light emission probability density $p^{doi}(x)$ with respect to each position x in the scintillator, on the basis of the value of the attenuation coefficient $\mu$. Subsequently, by employing the calculating function 150b, the processing circuitry 150 is configured to calculate the light emission distribution function model $p^{i,doi}_{ph;m}(t)$ considering the light emission position uncertainty and determined for each coincidence event, on the basis of the distribution function model $p^i_{ph;m}(t)$ determined for each coincidence event and the light emission probability density $p^{doi}(x)$. After that, by employing the specifying function 150c, the processing circuitry 150 is configured to calculate the light emission probability model $p^{i,doi}_{t;m}(t)$ considering the light emission position uncertainty and determined for each coincidence event, on the basis of the light emission distribution function model $p^{i,doi}_{ph;m}(t)$ considering the light emission position uncertainty and determined for each coincidence event.

As explained above, by employing the specifying function 150c, the processing circuitry 150 is configured to calculate the second light emission probability model $p_{t,m}(t)$ determined for each coincidence event, while taking the uncertainty of the light emission position in the scintillator into consideration.

Next, returning to the description of FIG. 13, at step S500, by employing the specifying function 150c, the processing circuitry 150 specifies a probability distribution model $p^{i,(doi)}_{ct;l,m}(\theta_l, \theta_m, t)$ that is related to the coincidence defined based on the first event and the second event and specified for each coincidence event, on the basis of the first light emission probability model $p^{doi}_{t;l}(t_1)$ calculated at step S300 and the second light emission probability model $p^{doi}_{t;m}(t_2)$.

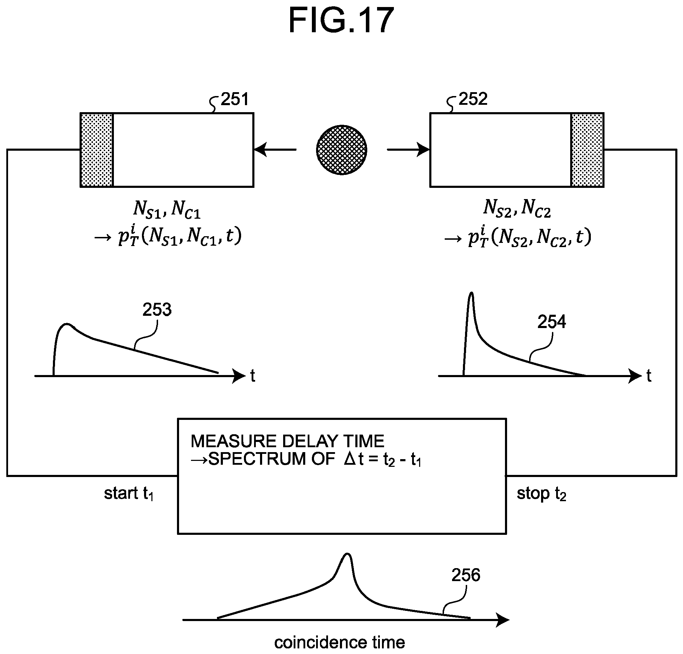

In FIG. 17, a curve 253 expresses a result of plotting the first light emission probability model $p^{i,doi}_{t;l}(\theta_l, t_l)$ determined for each coincidence event at a scintillator 251 of the first detector l, as a mathematical function of the delay time $t_l$. Also, another curve 254 expresses a result of plotting the second light emission probability model $p^{i,doi}_{t;m}(\theta_m, t_m)$ at a scintillator 252 of the second detector m as a mathematical function of the delay time $t_m$. By employing the specifying function 150c, the processing circuitry 150 is configured to specify the probability distribution model $p^{i,doi}_{ct;l,m}(\theta_l, \theta_m, t)$ which is determined for each coincidence event and expresses the event in the detector l at the detection time $t_l$ and an intersection of events in the detector m at the detection time $t_m$, as a mathematical function of the detection time difference. Yet another curve 256 expresses an example of the probability distribution model $P^{i,doi}_{ct;l,m}(\theta_l, \theta_m, t)$ specified in this manner.

In this situation, for the duration of a time width dt, it is possible to express the number of the events in the detector l at the detection time $t_l$ and the number of the intersections of events in the detector m at the detection time $t_m$, by using Expression (36) presented below.

$$p^{i,(doi)}_{t;l}\big(\theta^i_l,\ t_l\big)p^{i(doi)}_{t;m}\big(\theta^i_m, t_m\big)dt \qquad \cdots (36)$$

In the above expression, $\theta^i_l$ denotes a parameter set obtained at the detector l with respect to an i-th coincidence event. Further, when the parameter $\theta$ is expressed in a more specific form, Expression (37) presented below holds true.

$$p^{i,(doi)}_{t;l}\big(N^i_{s;l}, N^i_{c;l},\ t_l\big)p^{i(doi)}_{t;m}\big(N^i_{s;m}, N^i_{c;m}, t_m\big)dt \qquad \cdots (37)$$

In the above expression, $N^i_{s;l}$ and $N^i_{s;m}$ denote scintillation photon quantities at the detectors l and m related to the i-th coincidence event. $N^i_{c;l}$ and $N^i_{c;m}$ denote Cherenkov photon quantities at the detectors 1 and m related to the i-th coincidence event.

Further, it is possible to express the detection time difference between the detection time $t_l$ of the detector 1 and the detection time $t_m$ of the detector m, by using Expression (28) previously presented.

Consequently, it is possible to express $p^{i,doi}_{ct;l,m}(\theta_l,\theta_m,t)$ by using Expression (38) presented below.

$$p^{i,(doi)}_{ct;l,m}(\theta^i_l, \theta^i_m, t) = \int_0^\infty p^{i,(doi)}_{t;l}(\theta^i_l, t')p^{i,(doi)}_{t;m}(\theta^i_m, t' - t)dt' \qquad \cdots (38)$$

Further, when the parameter θ is expressed in a more specific form, Expression (39) presented below holds true.

$$\begin{aligned} p^{i,(doi)}_{ct;l,m}&\left(N^i_{s;l}, N^i_{c;l}, N^i_{s;m}, N^i_{c;m}, t\right) \\ &= \int_0^\infty p^{i,(doi)}_{t;l}\left(N^i_{s;l}, N^i_{c;l}, t'\right)p^{i,(doi)}_{t;m}\left(N^i_{s;m}, N^i_{c;m}, t' - t\right)dt' \end{aligned} \qquad \cdots (39)$$

As explained above, by employing the specifying function 150c and performing the process at step S30 in FIG. 13, the processing circuitry 150 is configured to calculate the probability distribution model $p^{i,doi}_{ct;l,m}(\theta^i_l,\theta^i_m,t)$ related to the detection time difference along the LOR defined by the first detector 1 and the second detector m, with respect to the i-th coincidence event. By performing the calculating process with respect to each of all the coincidence events and adding up the results, the processing circuitry 150 is able to calculate a final probability distribution model $p^{doi}_{ct;l,m}(\theta^i_l,\theta^i_m,t)$ related to the detection time difference along the LOR defined by the first detector 1 and the second detector m and to reconstruct the data on the basis of the calculated model.

At step S40, by employing the specifying function 150c, the processing circuitry 150 is configured to judge whether or not the process at step S30 has been finished with respect to each of all the coincidence events. When the processing circuitry 150 determines, by employing the specifying function 150c, that the process at step S30 has not been finished with respect to each of all the coincidence events (step S40: No), the process proceeds to step S50, where the process is performed on an (i+1)-th data. On the contrary, when the processing circuitry 150 determines, by employing the specifying function 150c, that the process at step S30 has been finished with respect to each of all the coincidence events (step S40: Yes), the process proceeds to step S60.

At step S60, by employing the specifying function 150c, the processing circuitry 150 generates a probability distribution model $p^{tot}_{ct;l,m}$ related to the coincidence at the first detector 1 and the second detector m, by adding up the probability distribution models $p^{i,doi}_{ct;l,m}(\theta^i_l,\theta^i_m,t)$ each of which corresponds to a different one of the coincidence events and was calculated at step S30.

More specifically, it is possible to express the probability distribution model $p^{tot}_{ct;l,m}$ related to the coincidence at the first detector 1 and the second detector m, by using Expression (41) presented below.

$$p^{tot}_{ct;l,m}(t) = \sum_i p^{i,(doi)}_{ct;l,m}(\theta^i_l, \theta^i_m, t) \qquad \cdots (41)$$

Further, the probability distribution model $p^{tot}_{ct;l,m}$ related to the coincidence at the first detector 1 and the second detector m, is used for an image reconstruction as being used as a ToF kernel at step S70 (explained later). In other words, by employing the specifying function 150c, the processing circuitry 150 is configured to generate the probability distribution model $p^{tot}_{ct;l,m}$ related to the coincidence at the first detector 1 and the second detector m, as the ToF kernel between the first detector 1 and the second detector m, by adding up, with respect to the events, the probability distribution models $p^{i,doi}_{ct;l,m}(\theta^i_l,\theta^i_m,t)$ each of which was specified for a different one of the events. In this manner, by employing the specifying function 150c, the processing circuitry 150 is configured, on the basis of the first photon number information and the second photon number information, to specify the probability distribution model $p^{tot}_{ct;l,m}$ related to the coincidence defined based on the first event and the second event.

In this situation, in the second embodiment, by employing the specifying function 150c, the processing circuitry 150 is configured to generate the probability distribution model $p^{i,doi}_{ct;l,m}(\theta^i_l,\theta^i_m,t)$ for each coincidence event at step S30 and to subsequently generate the final probability distribution model $p^{tot}_{ct;l,m}$ by adding up the generated models at step S60. Accordingly, by employing the specifying function 150c, the processing circuitry 150 is able to generate the ToF kernel that takes into consideration the variation of each event from an average of the measurement values. Consequently, even when there are a small number of events that make the variation in the measurement seem large, for example, the processing circuitry 150 is able to generate the valid ToF kernel by employing the specifying function 150c.

Subsequently, at step S70, by employing the reconstructing function 150d, the processing circuitry 150 reconstructs data corresponding to the LOR between the first detector 1 and the second detector m, by using, as a ToF kernel, the probability distribution model $p^{tot}_{ct;l,m}$ related to the coincidence defined based on the first photon number information and the second photon number information and specified at step S60.

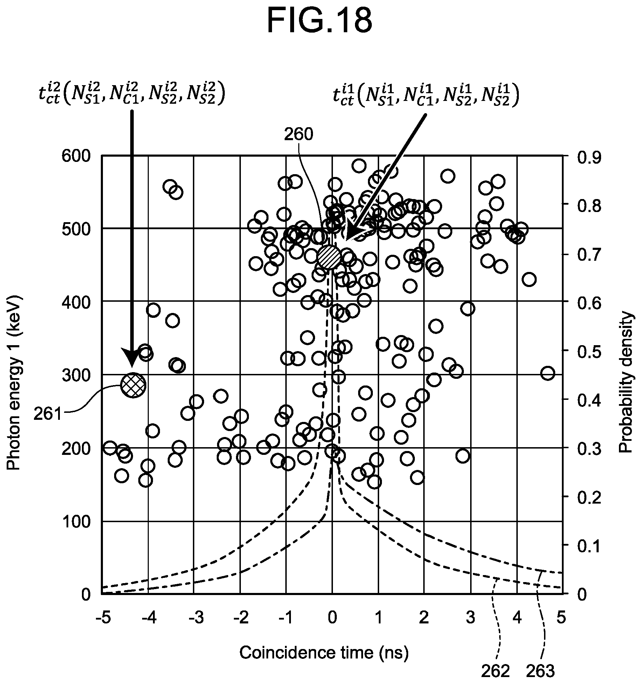

FIGS. 18 to 20 illustrate a result verifying the method according to the second embodiment. In this verification, the probability distribution model $p^{tot}_{ct;l,m}$ was generated from list mode data by using the present method and was compared with actual measurement values.

FIG. 18 illustrates a scatter plot in a list mode measured by using a BGO crystal. Each of the dots (e.g., an event 260, an event 261) in the chart corresponds to one event. By employing the calculating function 150b, the processing circuitry 150 calculated an event-specific probability distribution model $p_{ct}(t)$ corresponding to the event 260, on the basis of the event 260. A curve 263 represents the model $p_{ct}(t)$ corresponding to the event 260. Further, by employing the calculating function 150b, the processing circuitry 150 calculated another event-specific probability distribution model $p_{ct}(t)$ corresponding to the event 261 on the basis of the event 261. A curve 262 represents the model $p_{ct}(t)$ corresponding to the event 261. By employing the calculating function 150b, the processing circuitry 150 performed the same process with respect to each of all the events and thus generated a probability distribution model $p^{tot}(t)$ by adding up the probability distribution models $p_{ct}(t)$ each of which corresponded to a different one of the events.

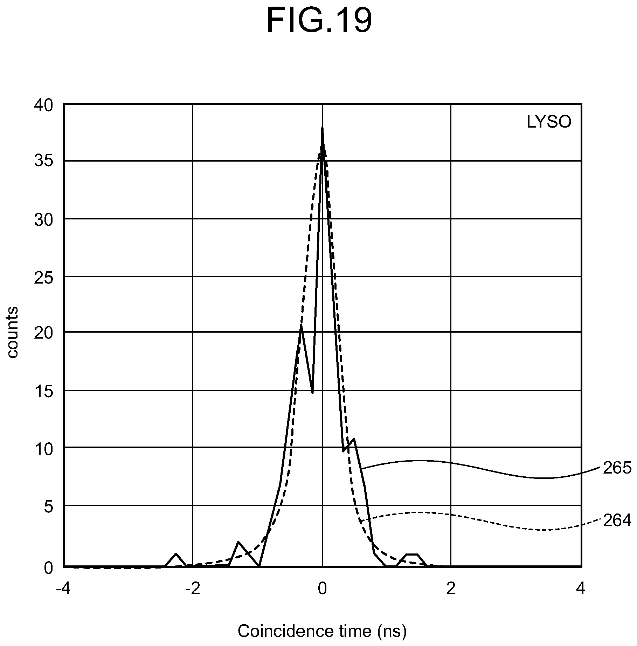

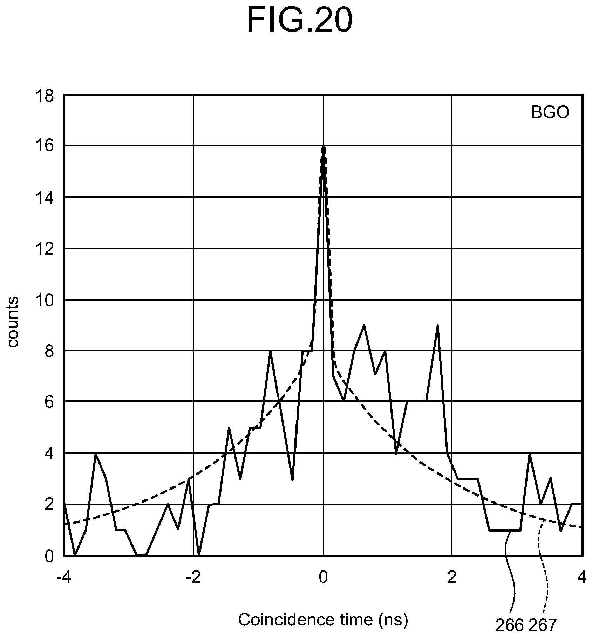

FIG. 19 illustrates comparison between actual measurement values using LYSO and the probability distribution model $p^{tot}_{ct}(t)$. A curve 265 represents the actual measurement values, whereas another curve 264 represents the calculated probability distribution model $p^{tot}_{ct}(t)$. Further, FIG. 20 illustrates comparison between actual measurement values using BGO and the probability distribution model $p^{tot}_{ct}(t)$. A curve 266 represents the actual measurement values, whereas another curve 267 represents the calculated probability distribution model $p^{tot}_{ct}(t)$. In both examples, the actual measurement values substantially matched the probability distribution models $p^{tot}(t)$. We were thus able to confirm validity of the method according to the second embodiment by which the probability distribution model $p_{ct}(t)$ was generated on the basis of the light emission model with respect to each coincidence event and the probability distribution model $p^{tot}_{ct}(t)$ was further generated by adding up the generated models.

Possible embodiments are not limited to the examples described above. Although the examples were explained in which the detectors 3 are ordinary detectors and do not structure a monolithic detector, possible embodiments are not limited to those examples. The embodiments are also applicable to the situation where a detector 3 is a monolithic scintillator structured as a single crystal, so that the single scintillator crystal is used in an integral form without being divided. The scintillator is formed by using the scintillator crystal of Lutetium Yttrium Oxyorthosilicate (LYSO), Lutetium Oxyorthosilicate (LSO), Lutetium Gadolinium Oxyorthosilicate (LGSO), or the like, for example.

When the detector 3 is a monolithic detector, the detector 3 is configured so that the single scintillator crystal is used in the integral form without being divided; however, on the basis of the concept where the detector 3 is structured with a plurality of virtual detectors, it is possible to perform the same processes by generating a ToF kernel corresponding to each set of virtual detectors. For example, when the detector 3 includes a plurality of detecting element arrays, it is possible to perform the same processes as those in the flowchart of FIG. 13, by considering each of the detecting element arrays as one detector. In an example, by performing the same processes while replacing the numbers assigned to the detectors with numbers assigned to the detecting element arrays, regarding the natural numbers 1 and m used at steps S10 through S70 in FIG. 13, steps S100 through S300 in FIG. 14, and in Expressions (35), (38), and so on, the processing circuitry 150 is able, by the specifying function, to specify a probability distribution model related to a detection time difference corresponding to the set of detecting element arrays.

In other words, when the detector 3 is a monolithic detector, a nuclear medicine diagnosis apparatus according to the second embodiment includes the processing circuitry 150. By employing the obtaining function 150a, the processing circuitry 150 is configured to obtain the monolithic detector including at least the first detecting element array 1 and the second detecting array m, the first photon number information corresponding to the first event detected by the first detecting element array 1 and the second photon number information corresponding to the first event and detected by the second detecting element array m. By employing the specifying function 150c, the processing circuitry 150 is configured to specify the probability distribution model $p^{tot}_{ct;l,m}$ related to the detection time difference corresponding to the set made up of the first detecting element array 1 and the second detecting element array m, on the basis of the first photon number information and the second photon number information.

Further, a data processing method according to the second embodiment includes: measuring, with respect to the monolithic detector including at least the first detecting element array 1 and the second detecting element array m, the first photon number information corresponding to the first event detected by the first detecting element array 1 and the second photon number information corresponding to the first event and detected by the second detecting element array m; and specifying the probability distribution model $p^{tot}_{ct;l,m}$ related to the detection time difference corresponding to the set made up of the first detecting element array 1 and the second detecting element array m, on the basis of the first photon number information and the second photon number information.

Further, a program according to the second embodiment causes a computer to execute: measuring, with respect to the monolithic detector including at least the first detecting element array 1 and the second detecting element array m, the first photon number information corresponding to the first event detected by the first detecting element array 1 and the second photon number information corresponding to the first event and detected by the second detecting element array m; and specifying the probability distribution model $p^{tot}_{ct;l,m}$ related to the detection time difference corresponding to the set made up of the first detecting element array 1 and the second detecting element array m, on the basis of the first photon number information and the second photon number information.

As explained above, in the nuclear medicine diagnosis apparatus according to the second embodiment, the processing circuitry 150 is configured to calculate, with respect to each coincidence event, the probability distribution model related to the detection time difference on the basis of the light emission model in the scintillator and to further generate the probability distribution model related to the detection time difference by adding up the calculated models. Accordingly, it is possible to perform the image reconstruction by generating the ToF kernel that takes into consideration the variation of each event from an average of the measurement values. Consequently, even when there are a small number of events that make the variation in the measurement seem large, for example, it is possible to enhance image quality of the PET image resulting from the reconstruction.

According to at least one aspect of the embodiments described above, it is possible to enhance the image quality.

## Claims

1. A nuclear medicine diagnosis apparatus (100) comprising processing circuitry (150) configured:

   to obtain first photon number information detected by a first detector (3) and second photon number information detected by a second detector (3) different from the first detector (3);
   to calculate a first light emission probability model corresponding to the first detector (3) on a basis of the first photon number information and a second light emission probability model corresponding to the second detector (3) on a basis of the second photon number information; and
   to specify a probability distribution model related to a detection time difference along a Line Of Response (LOR) defined by the first detector (3) and the second detector (3), on a basis of the first light emission probability model and the second light emission probability model.

2. The nuclear medicine diagnosis apparatus (100) according to claim 1, wherein the processing circuitry (150) is configured to reconstruct data corresponding to the LOR, by using the probability distribution model related to the detection time difference as a Time Of Flight (ToF) kernel.

3. The nuclear medicine diagnosis apparatus (100) according to claim 1 or 2, wherein the first light emission probability model and the second light emission probability model are each a model related to the probability density for detecting a first photon among a plurality of photons included in a detection event.

4. The nuclear medicine diagnosis apparatus (100) according to any one of claims 1 to 3, wherein the first light emission probability model and the second light emission probability model are each a model related to the probability density for detecting a prescribed number of photons among a plurality of photons included in a detection event.

5. The nuclear medicine diagnosis apparatus (100) according to any one of claims 1 to 4, wherein the first photon number information and the second photon number information are specified by performing a prescribed calibration scan.

6. The nuclear medicine diagnosis apparatus (100) according to claim 5, wherein the first photon number information and the second photon number information are specified on a basis of energy spectra obtained by the first detector and the second detector, respectively, by performing the calibration scan.

7. The nuclear medicine diagnosis apparatus (100 according to claim 1, wherein the first light emission probability model and the second light emission probability model are based on light emission distribution function models at the first detector (3) and at the second detector (3), respectively.

8. The nuclear medicine diagnosis apparatus (100) according to claim 1, wherein the first light emission probability model and the second light emission probability model are based on expected photon number values corresponding to a detection event.

9. The nuclear medicine diagnosis apparatus (100) according to any one of claims 1 to 8, wherein the first photon number information and the second photon number information each include a scintillation photon number.

10. The nuclear medicine diagnosis apparatus (100) according to claim 9, wherein the first photon number information and the second photon number information each further include a Cherenkov photon number.

11. The nuclear medicine diagnosis apparatus (100) according to any one of claims 1 to 10, wherein the processing circuitry (150) is configured to calculate the first light emission probability model and the second light emission probability model, while taking uncertainty of a light emission position in a scintillator (11) into consideration.

12. The nuclear medicine diagnosis apparatus (100) according to claim 11, wherein

the processing circuitry (150) is configured to calculate light emission probability density for each of positions in the scintillator (11) on a basis of an attenuation coefficient, and
the processing circuitry (150) is configured to calculate the first light emission probability model and the second light emission probability model on a basis of a light emission distribution function and the light emission probability density.

13. The nuclear medicine diagnosis apparatus (100) according to claim 1, wherein

the processing circuitry (150) is configured to obtain the first photon number information related to a first event detected by the first detector (3) and the second photon number information related to a second event detected by the second detector (3), and
the processing circuitry (150) is configured to specify the probability distribution model related to coincidence defined based on the first event and the second event, on the basis of the first photon number information and the second photon number information.

14. The nuclear medicine diagnosis apparatus (100) according to claim 13, wherein the processing circuitry (150) is configured to reconstruct data corresponding to the Line Of Response (LOR) between the first detector (3) and the second detector (3), by using a probability distribution model related to the coincidence as a Time Of Flight (ToF) kernel.

15. The nuclear medicine diagnosis apparatus (100) according to claim 13, wherein the processing circuitry (150) is configured to generate a ToF kernel between the first detector and the second detector, by adding up two or more of the probability distribution models each of which is specified for a different one of the events.

16. The nuclear medicine diagnosis apparatus (100) according to claim 13, wherein

the processing circuitry (150) is configured to generate the first light emission probability model about the first event on a basis of the first photon number information and to generate the second light emission probability model about the second event on a basis of the second photon number information, and
the processing circuitry (150) is configured to specify the probability distribution model on the basis of the first light emission probability model and the second light emission probability model.

17. The nuclear medicine diagnosis apparatus (100) according to claim 16, wherein the processing circuitry (150) is configured to generate the first light emission probability model and the second light emission probability model, on a basis of a light emission distribution function model determined for each of the events.

18. The nuclear medicine diagnosis apparatus (100) according to claim 13, wherein the first photon number information and the second photon number information being measured are obtained with respect to each of the events.

19. A data processing method for a nuclear medicine diagnosis apparatus (100) comprising:

obtaining, by processing circuitry (150), first photon number information detected by a first detector (3) and second photon number information detected by a second detector (3) different from the first detector (3);
calculating, by the processing circuitry (150), a first light emission probability model corresponding to the first detector (3) on a basis of the first photon number information and a second light emission probability model corresponding to the second detector (3) on a basis of the second photon number information;
specifying, by the processing circuitry (150), a probability distribution model related to a detection time difference along a Line Of Response (LOR) defined by the first detector (3) and the second detector (3), on a basis of the first light emission probability model and the second light emission probability model; and

reconstructing, by the processing circuitry (150), data corresponding to the LOR, by using the probability distribution model related to the detection time difference as a Time Of Flight (ToF) kernel.

20. A program for causing a processing circuitry (150) to execute the method of claim 19.

**Patentansprüche**

1. Nuklearmedizinische Diagnoseeinrichtung (100), die eine Verarbeitungsschaltung (150) umfasst, die konfiguriert ist:

    erste Photonenzahlinformationen, die von einem ersten Detektor (3) detektiert werden, und zweite Photonenzahlinformationen, die von einem zweiten Detektor (3) detektiert werden, der sich vom ersten Detektor (3) unterscheidet, zu erhalten;
    ein erstes Lichtemissionswahrscheinlichkeitsmodell entsprechend dem ersten Detektor (3) auf Basis der ersten Photonenzahlinformationen und ein zweites Lichtemissionswahrscheinlichkeitsmodell entsprechend dem zweiten Detektor (3) auf Basis der zweiten Photonenzahlinformationen zu berechnen; und
    ein Wahrscheinlichkeitsverteilungsmodell in Zusammenhang mit einer Detektionszeitdifferenz entlang einer durch den ersten Detektor (3) und den zweiten Detektor (3) definierten Antwortlinie (LOR) auf Basis des ersten Lichtemissionswahrscheinlichkeitsmodells und des zweiten Lichtemissionswahrscheinlichkeitsmodells zu spezifizieren.

2. Nuklearmedizinische Diagnoseeinrichtung (100) nach Anspruch 1, wobei die Verarbeitungsschaltung (150) konfiguriert ist, um Daten, die der LOR entsprechen, unter Verwendung des Wahrscheinlichkeitsverteilungsmodells in Zusammenhang mit der Detektionszeitdifferenz als Flugzeit-(ToF)-Kernel zu rekonstruieren.

3. Nuklearmedizinische Diagnoseeinrichtung (100) nach Anspruch 1 oder 2, wobei das erste Lichtemissionswahrscheinlichkeitsmodell und das zweite Lichtemissionswahrscheinlichkeitsmodell jeweils ein Modell sind, das mit der Wahrscheinlichkeitsdichte zum Detektieren eines ersten Photons aus einer Vielzahl von Photonen, die in einem Detektionsereignis eingeschlossen sind, zusammenhängt.

4. Nuklearmedizinische Diagnoseeinrichtung (100) nach einem der Ansprüche 1 bis 3, wobei das erste Lichtemissionswahrscheinlichkeitsmodell und das zweite Lichtemissionswahrscheinlichkeitsmodell jeweils ein Modell sind, das mit der Wahrscheinlichkeitsdichte zum Detektieren einer vorgeschriebenen Photonenzahl aus einer Vielzahl von Photonen, die in einem Detektionsereignis eingeschlossen sind, zusammenhängt.

5. Nuklearmedizinische Diagnoseeinrichtung (100) nach einem der Ansprüche 1 bis 4, wobei die ersten Photonenzahlinformationen und die zweiten Photonenzahlinformationen durch Durchführen eines vorgeschriebenen Kalibrierungsscans spezifiziert werden.

6. Nuklearmedizinische Diagnoseeinrichtung (100) nach Anspruch 5, wobei die ersten Photonenzahlinformationen und die zweiten Photonenzahlinformationen auf Basis von Energiespektren, die durch den ersten Detektor bzw. den zweiten Detektor durch Durchführen eines Kalibrierungsscans erhalten werden, spezifiziert werden.

7. Nuklearmedizinische Diagnoseeinrichtung (100) nach Anspruch 1, wobei das erste Lichtemissionswahrscheinlichkeitsmodell und das zweite Lichtemissionswahrscheinlichkeitsmodell auf Lichtemissionsverteilungsfunktionsmodellen am ersten Detektor (3) bzw. am zweiten Detektor (3) basieren.

8. Nuklearmedizinische Diagnoseeinrichtung (100) nach Anspruch 1, wobei das erste Lichtemissionswahrscheinlichkeitsmodell und das zweite Lichtemissionswahrscheinlichkeitsmodell auf erwarteten Photonenzahlenwerten basieren, die einem Detektionsereignis entsprechen.

9. Nuklearmedizinische Diagnoseeinrichtung (100) nach einem der Ansprüche 1 bis 8, wobei die ersten Photonenzahlinformationen und die zweiten Photonenzahlinformationen jeweils eine Szintillationsphotonenzahl beinhalten.

10. Nuklearmedizinische Diagnoseeinrichtung (100) nach Anspruch 9, wobei die ersten Photonenzahlinformationen und die zweiten Photonenzahlinformationen jeweils ferner eine Cherenkov-Photonenzahl beinhalten.

11. Nuklearmedizinische Diagnoseeinrichtung (100) nach einem der Ansprüche 1 bis 10, wobei die Verarbeitungsschaltung (150) konfiguriert ist, um das erste Lichtemissionswahrscheinlichkeitsmodell und das zweite Lichtemissionswahrscheinlichkeitsmodell unter Berücksichtigung einer Unsicherheit einer Lichtemissionsposition in einem Szintillator (11) zu berechnen.

12. Nuklearmedizinische Diagnoseeinrichtung (100) nach Anspruch 11, wobei

die Verarbeitungsschaltung (150) konfiguriert ist, um eine Lichtemissionswahrscheinlichkeitsdichte für jede Position im Szintillator (11) auf Basis eines Dämpfungskoeffizienten zu berechnen, und
die Verarbeitungsschaltung (150) konfiguriert ist, um das erste Lichtemissionswahrscheinlichkeitsmodell und das zweite Lichtemissionswahrscheinlichkeitsmodell auf Basis einer Lichtemissionsverteilungsfunktion und der Lichtemissionswahrscheinlichkeitsdichte zu berechnen.

13. Nuklearmedizinische Diagnoseeinrichtung (100) nach Anspruch 1, wobei

die Verarbeitungsschaltung (150) konfiguriert ist, um die ersten Photonenzahlinformationen in Zusammenhang mit einem ersten Ereignis, das vom ersten Detektor (3) detektiert wurde, und die zweiten Photonenzahlinformationen in Zusammenhang mit einem zweiten Ereignis, das vom zweiten Detektor (3) detektiert wurde, zu erhalten, und
die Verarbeitungsschaltung (150) konfiguriert ist, um das Wahrscheinlichkeitsverteilungsmodell in Zusammenhang mit Koinzidenz, die auf Basis des ersten Ereignisses und des zweiten Ereignisses definiert ist, auf Basis der ersten Photonenzahlinformationen und der zweiten Photonenzahlinformationen spezifiziert.

14. Nuklearmedizinische Diagnoseeinrichtung (100) nach Anspruch 13, wobei die Verarbeitungsschaltung (150) konfiguriert ist, um Daten zu rekonstruieren, die der Antwortlinie (LOR) zwischen dem ersten Detektor (3) und dem zweiten Detektor (3) entsprechen, indem sie ein Wahrscheinlichkeitsverteilungsmodell, das mit der Koinzidenz zusammenhängt, als Flugzeit-(ToF)-Kernel verwendet.

15. Nuklearmedizinische Diagnoseeinrichtung (100) nach Anspruch 13, wobei die Verarbeitungsschaltung (150) konfiguriert ist, um einen ToF-Kernel zwischen dem ersten Detektor und dem zweiten Detektor zu erzeugen, indem sie zwei oder mehrere der Wahrscheinlichkeitsverteilungsmodelle addiert, von denen jedes für ein anderes Ereignis spezifiziert ist.

16. Nuklearmedizinische Diagnoseeinrichtung (100) nach Anspruch 13, wobei

die Verarbeitungsschaltung (150) konfiguriert ist, um das erste Lichtemissionswahrscheinlichkeitsmodell für das erste Ereignis auf Basis der ersten Photonenzahlinformationen und das zweite Lichtemissionswahrscheinlichkeitsmodell für das zweite Ereignis auf Basis der zweiten Photonenzahlinformationen zu erzeugen, und
die Verarbeitungsschaltung (150) konfiguriert ist, um das Wahrscheinlichkeitsverteilungsmodell auf Basis des ersten Lichtemissionswahrscheinlichkeitsmodells und des zweiten Lichtemissionswahrscheinlichkeitsmodells zu spezifizieren.

17. Nuklearmedizinische Diagnoseeinrichtung (100) nach Anspruch 16, wobei die Verarbeitungsschaltung (150) konfiguriert ist, um auf Basis eines für jedes Ereignis ermittelten Lichtemissionsverteilungsfunktionsmodells das erste Lichtemissionswahrscheinlichkeitsmodell und das zweite Lichtemissionswahrscheinlichkeitsmodell zu erzeugen.

18. Nuklearmedizinische Diagnoseeinrichtung (100) nach Anspruch 13, wobei die ersten Photonenzahlinformationen und die zweiten Photonenzahlinformationen, die gemessen werden, in Bezug auf jedes der Ereignisse erhalten werden.

19. Datenverarbeitungsverfahren für eine nuklearmedizinische Diagnoseeinrichtung (100), umfassend:

Erhalten, durch eine Verarbeitungsschaltung (150), erster Photonenzahlinformationen, die von einem ersten Detektor (3) detektiert werden, und zweiter Photonenzahlinformationen, die von einem zweiten Detektor (3) detektiert werden, der sich vom ersten Detektor (3) unterscheidet;
Berechnen, durch die Verarbeitungsschaltung (150), eines ersten Lichtemissionswahrscheinlichkeitsmodells für den ersten Detektor (3) auf Basis der ersten Photonenzahlinformationen und eines zweiten Lichtemissionswahrscheinlichkeitsmodells für den zweiten Detektor (3) auf Basis der zweiten Photonenzahlinformationen;
Spezifizieren, durch die Verarbeitungsschaltung (150), eines Wahrscheinlichkeitsverteilungsmodells, das mit einer Detektionszeitdifferenz entlang einer durch den ersten Detektor (3) und den zweiten Detektor (3) definierten Antwortlinie (LOR) zusammenhängt, auf Basis des ersten Lichtemissionswahrscheinlichkeitsmodells und des zweiten Lichtemissionswahrscheinlichkeitsmodells; und

Rekonstruieren, durch die Verarbeitungsschaltung (150), von Daten, die der LOR entsprechen, unter Verwendung des Wahrscheinlichkeitsverteilungsmodells, das mit der Detektionszeitdifferenz zusammenhängt, als Flugzeit-(ToF)-Kernel.

20. Programm, das eine Verarbeitungsschaltung (150) veranlasst, das Verfahren nach Anspruch 19 auszuführen.

**Revendications**

1. Appareil de diagnostic de médecine nucléaire (100) comprenant un ensemble de circuits de traitement (150) configuré pour :

obtenir des premières informations sur le nombre de photons détectées par un premier détecteur (3) et des secondes informations sur le nombre de photons détectées par un second détecteur (3) différent du premier détecteur (3) ;

calculer un premier modèle de probabilité d'émission de lumière correspondant au premier détecteur (3) sur la base des premières informations sur le nombre de photons et un second modèle de probabilité d'émission de lumière correspondant au second détecteur (3) sur la base des secondes informations sur le nombre de photons ; et

spécifier un modèle de distribution de probabilité lié à une différence de temps de détection le long d'une ligne de réponse (LOR) définie par le premier détecteur (3) et le second détecteur (3), sur la base du premier modèle de probabilité d'émission de lumière et du second modèle de probabilité d'émission de lumière.

2. Appareil de diagnostic de médecine nucléaire (100) selon la revendication 1, dans lequel l'ensemble de circuits de traitement (150) est configuré pour reconstruire des données correspondant à la LOR, en utilisant le modèle de distribution de probabilité lié à la différence de temps de détection comme noyau de temps de vol (ToF).

3. Appareil de diagnostic de médecine nucléaire (100) selon la revendication 1 ou 2, dans lequel le premier modèle de probabilité d'émission de lumière et le second modèle de probabilité d'émission de lumière sont chacun un modèle lié à la densité de probabilité de détection d'un premier photon parmi une pluralité de photons inclus dans un événement de détection.

4. Appareil de diagnostic de médecine nucléaire (100) selon l'une quelconque des revendications 1 à 3, dans lequel le premier modèle de probabilité d'émission de lumière et le second modèle de probabilité d'émission de lumière sont chacun un modèle lié à la densité de probabilité de détection d'un nombre prescrit de photons parmi une pluralité de photons inclus dans un événement de détection.

5. Appareil de diagnostic de médecine nucléaire (100) selon l'une quelconque des revendications 1 à 4, dans lequel les premières informations sur le nombre de photons et les secondes informations sur le nombre de photons sont spécifiées en effectuant un balayage d'étalonnage prescrit.

6. Appareil de diagnostic de médecine nucléaire (100) selon la revendication 5, dans lequel les premières informations sur le nombre de photons et les secondes informations sur le nombre de photons sont spécifiées sur la base de spectres d'énergie obtenus respectivement par le premier détecteur et le second détecteur, en effectuant le balayage d'étalonnage.

7. Appareil de diagnostic de médecine nucléaire (100) selon la revendication 1, dans lequel le premier modèle de probabilité d'émission de lumière et le second modèle de probabilité d'émission de lumière sont basés sur des modèles de fonction de distribution d'émission de lumière au niveau du premier détecteur (3) et au niveau du second détecteur (3), respectivement.

8. Appareil de diagnostic de médecine nucléaire (100) selon la revendication 1, dans lequel le premier modèle de probabilité d'émission de lumière et le second modèle de probabilité d'émission de lumière sont basés sur des valeurs attendues du nombre de photons correspondant à un événement de détection.

9. Appareil de diagnostic de médecine nucléaire (100) selon l'une quelconque des revendications 1 à 8, dans lequel les premières informations sur le nombre de photons et les secondes informations sur le nombre de photons incluent toutes un nombre de photons de scintillation.

10. Appareil de diagnostic de médecine nucléaire (100) selon la revendication 9, dans lequel les premières informations sur le nombre de photons et les secondes informations sur le nombre de photons incluent toutes en outre un nombre de photons Cerenkov.

11. Appareil de diagnostic de médecine nucléaire (100) selon l'une quelconque des revendications 1 à 10, dans lequel l'ensemble de circuits de traitement (150) est configuré pour calculer le premier modèle de probabilité d'émission de lumière et le second modèle de probabilité d'émission de lumière, tout en prenant en considération l'incertitude d'une position d'émission de lumière dans un scintillateur (11).

12. Appareil de diagnostic de médecine nucléaire (100) selon la revendication 11, dans lequel

l'ensemble de circuits de traitement (150) est configuré pour calculer une densité de probabilité d'émission de lumière pour chacune des positions dans le scintillateur (11) sur la base d'un coefficient d'atténuation, et l'ensemble de circuits de traitement (150) est configuré pour calculer le premier modèle de probabilité d'émission de lumière et le second modèle de probabilité d'émission de lumière sur la base d'une fonction de distribution d'émission de lumière et de la densité de probabilité d'émission de lumière.

13. Appareil de diagnostic de médecine nucléaire (100) selon la revendication 1, dans lequel

l'ensemble de circuits de traitement (150) est configuré pour obtenir les premières informations sur le nombre de photons liées à un premier événement détecté par le premier détecteur (3) et les secondes informations sur le nombre de photons liées à un second événement détecté par le second détecteur (3), et l'ensemble de circuits de traitement (150) est configuré pour spécifier le modèle de distribution de probabilité lié à la coïncidence définie sur la base du premier événement et du second événement, sur la base des premières informations sur le nombre de photons et des secondes informations sur le nombre de photons.

14. Appareil de diagnostic de médecine nucléaire (100) selon la revendication 13, dans lequel l'ensemble de circuits de traitement (150) est configuré pour reconstruire des données correspondant à la ligne de réponse (LOR) entre le premier détecteur (3) et le second détecteur (3), en utilisant un modèle de distribution de probabilité lié à la coïncidence en tant que noyau de temps de vol (ToF).

15. Appareil de diagnostic de médecine nucléaire (100) selon la revendication 13, dans lequel l'ensemble de circuits de traitement (150) est configuré pour générer un noyau ToF entre le premier détecteur et le second détecteur, en additionnant jusqu'à deux des modèles de distribution de probabilité, ou plus, chacun étant spécifié pour un événement différent.

16. Appareil de diagnostic de médecine nucléaire (100) selon la revendication 13, dans lequel

l'ensemble de circuits de traitement (150) est configuré pour générer le premier modèle de probabilité d'émission de lumière concernant le premier événement sur la base des premières informations sur le nombre de photons et pour générer le second modèle de probabilité d'émission de lumière concernant le second événement sur la base des secondes informations sur le nombre de photons, et l'ensemble de circuits de traitement (150) est configuré pour spécifier le modèle de distribution de probabilité sur la base du premier modèle de probabilité d'émission de lumière et du second modèle de probabilité d'émission de lumière.

17. Appareil de diagnostic de médecine nucléaire (100) selon la revendication 16, dans lequel l'ensemble de circuits de traitement (150) est configuré pour générer le premier modèle de probabilité d'émission de lumière et le second modèle de probabilité d'émission de lumière, sur la base d'un modèle de fonction de distribution d'émission de lumière déterminé pour chacun des événements.

18. Appareil de diagnostic de médecine nucléaire (100) selon la revendication 13, dans lequel les premières informations sur le nombre de photons et les secondes informations sur le nombre de photons mesurées sont obtenues en rapport avec chacun des événements.

19. Procédé de traitement de données pour un appareil de diagnostic de médecine nucléaire (100) comprenant :

l'obtention, par un ensemble de circuits de traitement (150), de premières informations sur le nombre de photons détectées par un premier détecteur (3) et de secondes informations sur le nombre de photons détectées par un second détecteur (3), différent du premier détecteur (3) ;
le calcul, par l'ensemble de circuits de traitement (150), d'un premier modèle de probabilité d'émission de lumière correspondant au premier détecteur (3) sur la base des premières informations sur le nombre de photons et d'un second modèle de probabilité d'émission de lumière correspondant au second détecteur (3) sur la base des secondes informations sur le nombre de photons ;
la spécification, par l'ensemble de circuits de traitement (150), d'un modèle de distribution de probabilité lié à une différence de temps de détection le long d'une ligne de réponse (LOR) définie par le premier détecteur (3) et le second détecteur (3), sur la base du premier modèle de probabilité d'émission de lumière et du second modèle de probabilité d'émission de lumière ; et
la reconstruction, par l'ensemble de circuits de traitement (150), de données correspondant à la LOR, en utilisant le modèle de distribution de probabilité lié à la différence de temps de détection en tant que noyau de temps de vol (ToF).

20. Programme destiné à amener un ensemble de circuits de traitement (150) à exécuter le procédé de la revendication 19.

FIG.1

# FIG.2

START

S100

OBTAIN FIRST PHOTON NUMBER INFORMATION ($N_{s;l}, N_{c;l}$ ...) DETECTED BY FIRST DETECTOR $l$

S200

OBTAIN SECOND PHOTON NUMBER INFORMATION ($N_{s;l}, N_{c;l}$) DETECTED BY SECOND DETECTOR $m$

S300

CALCULATE FIRST LIGHT EMISSION PROBABILITY MODEL $p_{t;l}^{(doi)}(\boldsymbol{\theta}_l; t)$ CORRESPONDING TO FIRST DETECTOR $l$ ON THE BASIS OF FIRST PHOTON NUMBER INFORMATION

S400

CALCULATE SECOND LIGHT EMISSION PROBABILITY MODEL $p_{t;m}^{(doi)}(\boldsymbol{\theta}_m; t)$ CORRESPONDING TO SECOND DETECTOR $m$ ON THE BASIS OF SECOND PHOTON NUMBER INFORMATION

S500

SPECIFY PROBABILITY DISTRIBUTION MODEL $p_{ToF;l,m}^{(doi)}(t)$ RELATED TO DETECTION TIME DIFFERENCE BETWEEN LORs DEFINED BY FIRST DETECTOR AND SECOND DETECTOR, ON THE BASIS OF FIRST LIGHT EMISSION PROBABILITY MODEL $p_{t;l}^{(doi)}(\boldsymbol{\theta}_l; t)$ AND SECOND LIGHT EMISSION PROBABILITY MODEL $p_{t;m}^{(doi)}(\boldsymbol{\theta}_m; t)$

S600

RECONSTRUCT DATA CORRESPONDING TO LORs BY USING PROBABILITY DISTRIBUTION MODEL AS ToF KERNEL

END

# FIG.3

(1) HIGH ENERGY ELECTRON

EXCITATION      TIME $t$

$E_{th}$

(2) CHERENKOV LIGHT $n_c(t)$

$t_0$

$2\sigma_c$

TIME $t$

(3) EXCITATION DENSITY OF
VALENCE ELECTRON

$n_{ex}(t)$

$t_1$

$2\sigma_s$

$n_{ex}(t')\,dt'$        $t$    TIME $t$

(4) SCINTILLATION LIGHT $n_s(t)$

TIME $t$

# FIG.4

START

GENERATE LIGHT EMISSION DISTRIBUTION FUNCTION MODEL $p_{ph,l}(t)$ ON THE BASIS OF FIRST PHOTON NUMBER INFORMATION OF FIRST DETECTOR $l$ —— S310

CALCULATE FIRST LIGHT EMISSION PROBABILITY MODEL $p_{t;l}(\boldsymbol{\theta}_l; t)$ ON THE BASIS OF LIGHT EMISSION DISTRIBUTION FUNCTION MODEL $p_{ph,l}(t)$ —— S320A

END

# FIG.5

$p_{ph}(t)$

$N' = 4$

$N' = 3$

$N' = 2$

$N' = 1$

30

. . .

TIME $t$

# FIG.6

$p_t(t)$

31

TIME $t$

# FIG.7

LIGHT SPEED $c$

LIGHT PROPAGATION AT LIGHT SPEED $c/n$ IN SUBSTANCE

TIME $t_1$

TIME $t' = t_1 + t_2$

DEPTH 0 $x$ $L$

# FIG.8

START

S310

GENERATE LIGHT EMISSION DISTRIBUTION FUNCTION MODEL $p_{ph,l}(t)$ ON THE BASIS OF PHOTON NUMBER INFORMATION OF DETECTOR $l$

S311

CALCULATE LIGHT EMISSION PROBABILITY DENSITY $p_{doi}(x)$ WITH RESPECT TO EACH POSITION $x$, ON THE BASIS OF ATTENUATION COEFFICIENT $\mu$

S312

CALCULATE LIGHT EMISSION DISTRIBUTION FUNCTION MODEL $p_{ph;l}^{doi}(t)$ CONSIDERING LIGHT EMISSION POSITION UNCERTAINTY, ON THE BASIS OF DISTRIBUTION FUNCTION MODEL $p_{ph,l}(t)$ AND LIGHT EMISSION PROBABILITY DENSITY $p_{doi}(x)$

S320B

CALCULATE LIGHT EMISSION PROBABILITY MODEL $p_{t;l}^{doi}(\boldsymbol{\theta}_l; t)$ CONSIDERING LIGHT EMISSION POSITION UNCERTAINTY, ON THE BASIS OF LIGHT EMISSION DISTRIBUTION FUNCTION MODEL $p_{ph;l}^{doi}(t)$ CONSIDERING LIGHT EMISSION POSITION UNCERTAINTY

END

# FIG.9

$p_{t;l}^{(doi)}(\boldsymbol{\theta_l}; t_l)$

50

$t_l$

DELAY TIME

$p_{t;m}^{(doi)}(\boldsymbol{\theta_m}; t_m)$

51

$t_m$

DELAY TIME

$p_{ToF;l,m}^{(doi)}(\boldsymbol{\theta_l}, \boldsymbol{\theta_m}; t)$

52

DETECTION TIME DIFFERENCE $t = t_l - t_m$

EP 4 345 506 B1

# FIG.10

# FIG.11

# FIG.12

# FIG.13

START

SELECT DETECTOR (l-TH DETECTOR) TO
DETECT FIRST EVENT AND
DETECTOR (m-TH DETECTOR) TO
DETECT SECOND EVENT — S10

$i = 1$ — S20

CALCULATE PROBABILITY

DISTRIBUTION MODEL $p_{ct;l,m}^{i,(doi)}(\theta_l^i, \theta_m^i, t)$

RELATED TO COINCIDENCE WITH RESPECT
TO i-TH COINCIDENCE EVENT AMONG
COINCIDENCE EVENTS BETWEEN EVENTS
DETECTED BY l-TH DETECTOR AND
EVENTS DETECTED BY m-TH DETECTOR — S30

S40

HAVE ALL COINCIDENCE EVENTS
BEEN PROCESSED?

NO

S50

$i = i + 1$

YES

S60

GENERATE PROBABILITY DISTRIBUTION
MODEL $p_{ct;l,m}^{tot}(t)$ BY ADDING UP PROBABILITY

DISTRIBUTION MODELS $p_{ct;l,m}^{i,(doi)}(\theta_l^i, \theta_m^i, t)$

EACH CORRESPONDING TO
DIFFERENT ONE OF EVENTS

RECONSTRUCT DATA BY USING PROBABILITY
DISTRIBUTION MODEL $p_{ct;l,m}^{tot}(t)$
AS ToF KERNEL — S70

END

# FIG.14

START

S100

OBTAIN FIRST PHOTON NUMBER INFORMATION $\left(N_{s;l}^i, N_{c;l}^i \, \ldots\right)$
DETECTED BY FIRST DETECTOR I WITH RESPECT TO i-TH EVENT

S200

OBTAIN SECOND PHOTON NUMBER INFORMATION $\left(N_{s;m}^i, N_{c;m}^i \, \ldots\right)$
DETECTED BY SECOND DETECTOR m WITH RESPECT TO i-TH EVENT

S300

CALCULATE FIRST LIGHT EMISSION PROBABILITY MODEL $p_{T;l}^{i,(doi)}\left(\theta_l^i, t_l\right)$
CORRESPONDING TO FIRST DETECTOR I ON THE BASIS OF
FIRST PHOTON NUMBER INFORMATION

S400

CALCULATE SECOND LIGHT EMISSION PROBABILITY MODEL $p_{T;m}^{i,(doi)}\left(\theta_m^i, t_m\right)$
CORRESPONDING TO SECOND DETECTOR m ON THE BASIS OF
SECOND PHOTON NUMBER INFORMATION

S500

SPECIFY PROBABILITY DISTRIBUTION MODEL $p_{ct;l,m}^{i,(doi)}\left(\theta_l^i, \theta_m^i, t\right)$ RELATED
TO DETECTION TIME DIFFERENCE BETWEEN LORs DEFINED BY
FIRST DETECTOR AND SECOND DETECTOR, ON THE BASIS OF
FIRST LIGHT EMISSION PROBABILITY MODEL $p_{T;l}^{i,(doi)}\left(\theta_l^i, t_l\right)$

AND SECOND LIGHT EMISSION PROBABILITY MODEL $p_{T;m}^{i,(doi)}\left(\theta_m^i, t_m\right)$

END

# FIG.15

START

$\varsigma$ S310

GENERATE LIGHT EMISSION DISTRIBUTION FUNCTION MODEL $p^i_{ph;l}\left(\theta^i_l, t\right)$ WITH RESPECT TO i-TH EVENT, ON THE BASIS OF FIRST PHOTON NUMBER INFORMATION OF FIRST DETECTOR I

$\varsigma$ S320A

CALCULATE FIRST LIGHT EMISSION PROBABILITY MODEL $p^i_{T;l}\left(\theta^i_l, t\right)$, ON THE BASIS OF LIGHT EMISSION DISTRIBUTION FUNCTION MODEL $p^i_{ph;l}\left(\theta^i_l, t\right)$

END

# FIG.16

START

S310

GENERATE LIGHT EMISSION DISTRIBUTION FUNCTION MODEL $p_{ph;l}^{i}(\theta_{l}^{i}, t)$, ON THE BASIS OF PHOTON NUMBER INFORMATION OF DETECTOR I

S311

CALCULATE LIGHT EMISSION PROBABILITY DENSITY $p_{doi}(x)$ WITH RESPECT TO EACH POSITION x, ON THE BASIS OF ATTENUATION COEFFICIENT $\mu$

S312

CALCULATE LIGHT EMISSION DISTRIBUTION FUNCTION MODEL $p_{ph;l}^{doi}(t)$ CONSIDERING LIGHT EMISSION POSITION UNCERTAINTY, ON THE BASIS OF DISTRIBUTION FUNCTION MODEL $p_{ph;l}^{i}(\theta_{l}^{i}, t)$ AND LIGHT EMISSION PROBABILITY DENSITY $p_{doi}(x)$

S320B

CALCULATE LIGHT EMISSION PROBABILITY MODEL $p_{T;l}^{i,doi}(\theta_{l}^{i}, t)$ CONSIDERING LIGHT EMISSION POSITION UNCERTAINTY, ON THE BASIS OF LIGHT EMISSION DISTRIBUTION FUNCTION MODEL $p_{ph}^{i,doi}(t)$ CONSIDERING LIGHT EMISSION POSITION UNCERTAINTY

END

# FIG.17

251

252

$N_{S1}, N_{C1}$
$\rightarrow p_T^i(N_{S1}, N_{C1}, t)$

$N_{S2}, N_{C2}$
$\rightarrow p_T^i(N_{S2}, N_{C2}, t)$

253

254

t

t

MEASURE DELAY TIME
$\rightarrow$SPECTRUM OF $\Delta t = t_2 - t_1$

start $t_1$

stop $t_2$

256

coincidence time

# FIG.18

$$t_{ct}^{i2}\left(N_{S1}^{i2}, N_{C1}^{i2}, N_{S2}^{i2}, N_{S2}^{i2}\right) \qquad t_{ct}^{i1}\left(N_{S1}^{i1}, N_{C1}^{i1}, N_{S2}^{i1}, N_{S2}^{i1}\right)$$

# FIG.19

# FIG.20

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **EFTHIMIOU et al.** TOF-PET Image Reconstruction With Multiple Timing Kernels Applied on Cherenkov Radiation in BGO. *IEEE TRANSACTIONS ON RADIATION AND PLASMA MEDICAL SCIENCES*, September 2021, vol. 5 (5), 703-711